# EUROPEAN PATENT APPLICATION

(11) **EP 4 693 303 A1**
(43) Date of publication of application: **11.02.2026**
(21) Application number: 24778577.7
(22) Date of filing: 19.01.2024
(51) Int. Cl.: G16C 20/30, G06F 16/9038

(54) **INFORMATION PROCESSING DEVICE, INFORMATION PROCESSING METHOD, AND PROGRAM**

(30) Priority: 31.03.2023 JP 2023059422
(71) Applicant: FUJIFILM Corporation, Tokyo 106-8620 (JP)
(72) Inventor: TATESHITA, Masakazu, Ashigarakami-gun, Kanagawa 258-8577 (JP); HIKIDA, Yasushi, Ashigarakami-gun, Kanagawa 258-8577 (JP); MURAKAMI, Ryoichi, Ashigarakami-gun, Kanagawa 258-8577 (JP); SUGIYAMA, Satoshi, Ashigarakami-gun, Kanagawa 258-8577 (JP)
(74) Representative: HGF
(86) International application number: PCT/JP2024/001436
(87) International publication number: WO 2024/202431

(57) **Abstract**

An information processing apparatus includes a processor, in which the processor is configured to generate a display image that shows a prediction result of metabolic reactions occurring in vivo from a test substance and metabolites generated by the metabolic reactions and an evaluation result of toxicity of at least one compound in a compound group composed of the test substance and the metabolites, the display image showing the evaluation result of the compound in a distinguishable manner in a metabolic reaction pathway that systematically shows the metabolic reactions occurring in a chain starting from the test substance, and the resulting metabolites, and execute control of outputting the generated display image, and the metabolites systematically shown in the display image include a disappearance compound that disappears in the metabolic reaction pathway.

## Description

### BACKGROUND OF THE INVENTION

### 1. Field of the Invention

The technology of the present disclosure relates to an information processing apparatus, an information processing method, and a program.

### 2. Description of the Related Art

In recent years, in the field of handling chemical substances, a toxicity evaluation using a computer-based in silico method has become common. In the toxicity evaluation of chemical substances, it is necessary to evaluate not only a test substance to be tested but also toxicity of its metabolites in order to evaluate its toxicity to a living body.

WO2021/145434A discloses a method of predicting indications of a target drug or its equivalent substance, the method including: inputting estimated adverse event-related information estimated from a group of data showing behavior of biomarkers in one or more organs collected from a non-human animal to which the target drug or its equivalent substance has been administered as a test substance into a predictive artificial intelligence model as test data; and predicting the indications of the target drug or its equivalent substance.

WO2011/055820A discloses a support device comprising: a measurement data acquisition unit that acquires a plurality of measurement data obtained under different conditions for each of a plurality of types of metabolites and/or metabolic enzymes; a pathway map data acquisition unit that acquires data representing a metabolic pathway map within a living body; an association strength calculation unit that calculates strength of association between the metabolites and/or metabolic enzymes and each metabolic pathway based on the measurement data acquired by the measurement data acquisition unit; a display unit; and a display content determination unit that determines a content to be displayed on the display unit. In the support device, the display content determination unit can cause the display unit to display a content representing two or more metabolic pathways such that the strength of the association is distinguishable, the support device further comprises a pathway selection input receiving unit that receives a selection input of one of the metabolic pathways from a user in a case where the content representing the two or more metabolic pathways is displayed on the display unit, and the display content determination unit causes the display unit to display the metabolic pathway map for the metabolic pathway selected by the selection input received by the pathway selection input receiving unit, and determines the content to be displayed on the display unit such that an increase or decrease in measurement values indicated by the measurement value data for the metabolites and/or metabolic enzymes present on the metabolic pathway map is distinguishable.

JP2004-93234A discloses a toxicity presence/absence determination system including: a storage unit that stores a simultaneous differential equation based on gene pathway information related to metabolism or a sequence of a toxic substance, a reaction rate coefficient of the simultaneous differential equation, a relationship between linear stability and Jacobian stability according to the reaction rate coefficient, and a determination of presence or absence of toxicity of the toxic substance in a value of the reaction rate coefficient; an input unit that inputs gene expression distribution data to the simultaneous differential equation; and a display unit that obtains the reaction rate coefficient by inputting the gene expression distribution data to the simultaneous differential equation and that displays the determination of the presence or absence of the toxicity of the toxic substance.

### SUMMARY OF THE INVENTION

However, the technologies disclosed in WO2021/145434A, WO2011/055820A, and JP2004-93234A do not take into consideration how to display a prediction result of a metabolic reaction and an evaluation result of toxicity, and there is room for improvement in enabling the user to efficiently understand the prediction result and the evaluation result and supporting the user in evaluating toxicity of a compound.

One embodiment according to the technology of the present disclosure provides an information processing apparatus, an information processing method, and a program that enable a user to efficiently understand a prediction result of a metabolic reaction and an evaluation result of toxicity of a compound involved in the metabolic reaction, and that are capable of supporting the user in evaluating the toxicity of the compound.

A first aspect according to the technology of the present disclosure is an information processing apparatus comprising: a processor, in which the processor is configured to generate a display image that shows a prediction result of metabolic reactions occurring in vivo from a test substance and metabolites generated by the metabolic reactions and an evaluation result of toxicity of at least one compound in a compound group composed of the test substance and the metabolites, the display image showing the evaluation result of the compound in a distinguishable manner in a metabolic reaction pathway that systematically shows the metabolic reactions occurring in a chain starting from the test substance, and the resulting metabolites, and execute control of outputting the generated display image, and the metabolites systematically shown in the display image include a disappearance compound that disappears in the metabolic reaction pathway.

A second aspect according to the technology of the present disclosure is the information processing apparatus according to the first aspect, in which the evaluation result of the disappearance compound is displayed in the display image in a distinguishable manner from other metabolites.

A third aspect according to the technology of the present disclosure is the information processing apparatus according to the first aspect, in which the metabolic reaction pathway shown in the display image is a tree diagram shown by compound data representing each compound of the test substance and the metabolites and connecting lines connecting items of the compound data before and after the metabolic reaction.

A fourth aspect according to the technology of the present disclosure is the information processing apparatus according to the third aspect, in which a name of the metabolic reaction is displayed in association with the connecting line.

A fifth aspect according to the technology of the present disclosure is the information processing apparatus according to the third aspect, in which, in a case where the connecting line is selected, a chemical structure that causes the metabolic reaction is displayed, the chemical structure being a partial structure contained in the compound before the reaction.

A sixth aspect according to the technology of the present disclosure is the information processing apparatus according to the first aspect, in which, in the metabolic reaction pathway, in a case where partial metabolic reaction pathways originating from a plurality of the metabolites different from each other are shared, the partial metabolic reaction pathways are collectively displayed as one.

A seventh aspect according to the technology of the present disclosure is the information processing apparatus according to the first aspect, in which, in a case where the compound, which is a target of an evaluation of the toxicity and has the evaluation result, is selected in the metabolic reaction pathway in the display image, a determination flow indicating a toxicity determination procedure, which is used in the evaluation of the toxicity, has at least one determination step, and has a path that branches according to a determination result of the determination step, is displayed, in which, among a plurality of paths, a path that leads to the evaluation result of the compound is shown in a distinguishable manner from the other paths.

An eighth aspect according to the technology of the present disclosure is the information processing apparatus according to the first aspect, in which, in the display image, a display range of the metabolic reaction pathway is adjustable.

A ninth aspect according to the technology of the present disclosure is an information processing method comprising: generating a display image that shows a prediction result of metabolic reactions occurring in vivo from a test substance and metabolites generated by the metabolic reactions and an evaluation result of toxicity of at least one compound in a compound group composed of the test substance and the metabolites, the display image showing the evaluation result of the compound in a distinguishable manner in a metabolic reaction pathway that systematically shows the metabolic reactions occurring in a chain starting from the test substance, and the resulting metabolites; and executing control of outputting the generated display image, in which the metabolites systematically shown in the display image include a disappearance compound that disappears in the metabolic reaction pathway.

A tenth aspect according to the technology of the present disclosure is a program for causing a computer to execute a process comprising: generating a display image that shows a prediction result of metabolic reactions occurring in vivo from a test substance and metabolites generated by the metabolic reactions and an evaluation result of toxicity of at least one compound in a compound group composed of the test substance and the metabolites, the display image showing the evaluation result of the compound in a distinguishable manner in a metabolic reaction pathway that systematically shows the metabolic reactions occurring in a chain starting from the test substance, and the resulting metabolites; and executing control of outputting the generated display image, in which the metabolites systematically shown in the display image include a disappearance compound that disappears in the metabolic reaction pathway.

The technology of the present disclosure provides an information processing apparatus, an information processing method, and a program that enable a user to efficiently understand a prediction result of a metabolic reaction and an evaluation result of toxicity of a compound involved in the metabolic reaction, and that are capable of supporting the user in evaluating the toxicity of the compound.

### BRIEF DESCRIPTION OF THE DRAWINGS

FIG. 1 is a conceptual diagram showing a schematic configuration example of an information processing system.
FIG. 2 is a block diagram showing an example of a hardware configuration of an electrical system of a server.
FIG. 3 is a conceptual diagram showing an example of a usage aspect of the information processing system.
FIG. 4 is a functional block diagram showing an example of main functions of a processor of the server.
FIG. 5 is a conceptual diagram showing an example of processing contents of a toxicity evaluation unit.
FIG. 6 is a functional block diagram showing an example of main functions of the processor of the server.
FIG. 7 is a conceptual diagram showing an example of an aspect in which a display image is displayed on a display device.
FIG. 8 is a flowchart showing an example of a flow of server control processing.
FIG. 9 is a functional block diagram showing an example of main functions of the processor of the server.
FIG. 10 is a conceptual diagram showing an example of an aspect in which a display image is displayed on the display device.
FIG. 11 is a functional block diagram showing an example of main functions of the processor of the server.
FIG. 12 is a conceptual diagram showing an example of an aspect in which a display image is displayed on the display device.
FIG. 13 is a functional block diagram showing an example of main functions of the processor of the server.
FIG. 14 is a conceptual diagram showing an example of an aspect in which a display image is displayed on the display device.
FIG. 15 is a functional block diagram showing an example of main functions of the processor of the server.
FIG. 16 is a conceptual diagram showing an example of an aspect in which a display image is displayed on the display device.
FIG. 17 is a conceptual diagram showing an example of an aspect in which a display image is displayed on the display device.
FIG. 18 is a block diagram showing an example of a hardware configuration of an electrical system of a client terminal.
FIG. 19 is a functional block diagram showing an example of main functions of a processor of the client terminal.
FIG. 20 is a functional block diagram showing an example of main functions of the processor of the client terminal.

### DESCRIPTION OF THE PREFERRED EMBODIMENTS

An example of embodiments of an information processing apparatus, an information processing method, and a program according to the technology of the present disclosure will be described with reference to the accompanying drawings.

### [First Embodiment]

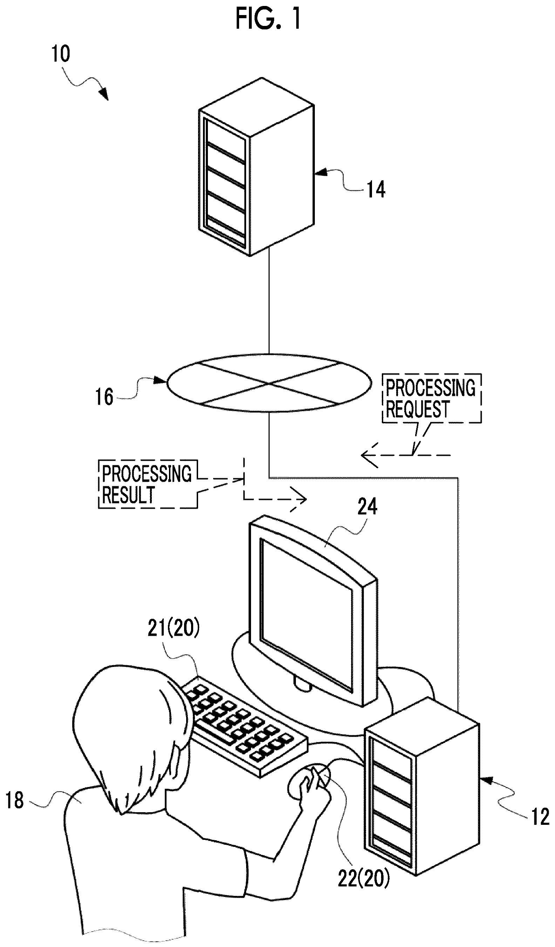

As shown in FIG. 1 as an example, an information processing system 10 comprises a client terminal 12 and a server 14. In the information processing system 10, the client terminal 12 and the server 14 are communicably connected to each other via a network 16.

The information processing system 10 is used, for example, for a prediction of a reaction (for example, a metabolic reaction in a body) related to a chemical substance and/or an evaluation of a characteristic of a chemical substance (for example, an evaluation of the presence or absence of toxicity to a human body). A user 18 (for example, a researcher) can use the information processing system 10 to predict and evaluate experimental results using computer simulations (that is, an evaluation using in silico method). For example, for a newly developed or under-development chemical substance, it is possible to obtain an evaluation result of toxicity without performing a toxicity evaluation test (that is, an in vitro or in vivo toxicity evaluation test) using an actual chemical substance.

The client terminal 12 is a terminal used by the user 18. The server 14 receives a processing request from the client terminal 12 via the network 16 and provides a service corresponding to the request to the client terminal 12 that has made the request via the network 16. For example, in a case where the server 14 receives a request to execute processing related to a reaction prediction and a characteristic evaluation of a chemical substance as the processing request, the server 14 transmits a prediction result and an evaluation result to the client terminal 12. In the present embodiment, the server 14 is an example of an "information processing apparatus" according to the technology of the present disclosure.

For example, the server 14 is realized by a mainframe, but this is merely an example. For example, the server may be realized by cloud computing, or may be realized by network computing such as fog computing, edge computing, or grid computing. Here, the server 14 is exemplified as an example of a device provided outside the client terminal 12, but this is merely an example, and at least one personal computer or the like may be used instead of the server 14.

The network 16 is configured by, for example, at least one of a wide area network (WAN) or a local area network (LAN). Further, a connection method between the client terminal 12 and the network 16 and a connection method between the server 14 and the network 16 may be a wireless communication method or a wired communication method. The network 16 establishes communication between the client terminal 12 and the server 14, and transmits and receives various types of information between the client terminal 12 and the server 14.

A reception device 20 is connected to the client terminal 12. The reception device 20 receives an instruction from the user 18. The reception device 20 includes a keyboard 21, a mouse 22, and the like. The keyboard 21 and the mouse 22 shown in FIG. 1 are merely an example. As the reception device 20, any one of the keyboard 21 or the mouse 22 may be provided. In addition, as the reception device 20, for example, at least one of a proximity input device that receives a proximity input, a voice input device that receives a voice input, or a gesture input device that receives a gesture input may be applied instead of the keyboard 21 and/or the mouse 22. The proximity input device is, for example, a touch panel or a tablet.

A display device 24 is connected to the client terminal 12. Examples of the display device 24 include an electro-luminescence (EL) display and a liquid crystal display. The display device 24 displays various types of information (for example, image and text) under the control of the client terminal 12.

Here, a personal computer is described as an example of the client terminal 12, but this is merely an example. As the client terminal 12, a mobile terminal, such as a smartphone and a tablet terminal, may be used. Here, although an example in which one client terminal 12 is connected to one server 14 via the network 16 has been described, this is merely an example. It is needless to say that the information processing system 10 may include a plurality of client terminals 12 and a plurality of servers 14.

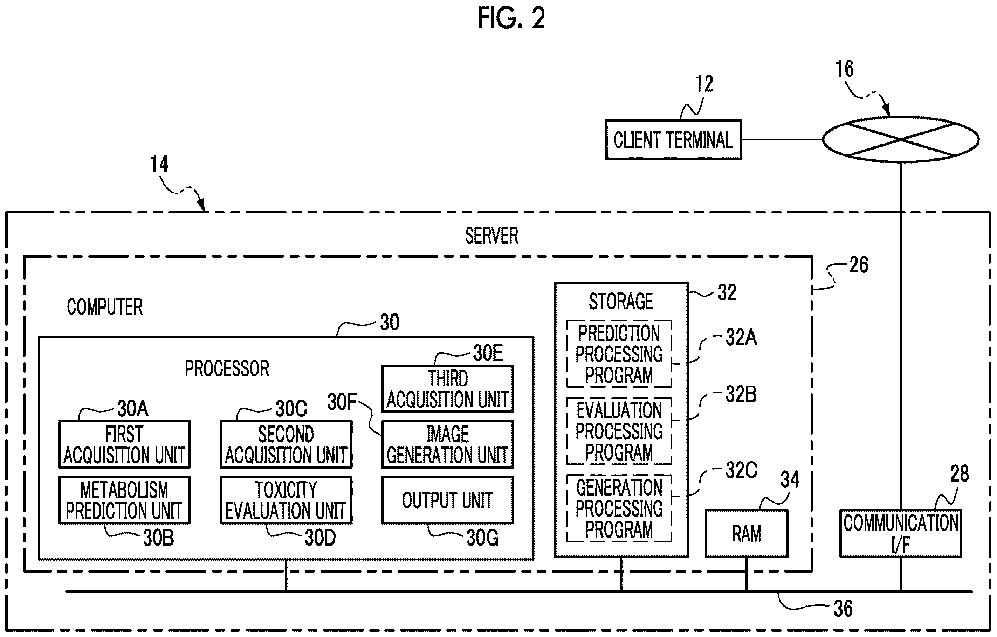

As shown in FIG. 2 as an example, the server 14 comprises a computer 26, a communication interface (I/F) 28, and a bus 36. The computer 26 comprises a processor 30, a storage 32, and a random access memory (RAM) 34. The processor 30, the storage 32, the RAM 34, and the communication I/F 28 are connected to the bus 36. The computer 26 is an example of a "computer" according to the technology of the present disclosure, and the processor 30 is an example of a "processor" according to the technology of the present disclosure.

A memory is connected to the processor 30. The memory includes the storage 32 and the RAM 34. The processor 30 includes, for example, a central processing unit (CPU) and a graphics processing unit (GPU). The GPU operates under the control of the CPU, and is responsible for executing processing related to an image.

The storage 32 is a non-volatile storage device that stores various programs, various parameters, and the like. Examples of the storage 32 include a flash memory (for example, an electrically erasable and programmable read only memory (EEPROM) and a solid state drive (SSD)), and/or a hard disk drive (HDD). The flash memory and the HDD are merely examples, and at least one of the flash memory, the HDD, a magnetoresistive memory, or a ferroelectric memory may be used as the storage 32.

The RAM 34 is a memory that transitorily stores information, and is used as a work memory by the processor 30. Examples of the RAM 34 include a dynamic random access memory (DRAM) or a static random access memory (SRAM).

The communication I/F 28 is connected to the network 16. The communication I/F 28 is responsible for transmitting and receiving information to and from an external communication device (for example, the client terminal 12) via the network 16. For example, the communication I/F 28 transmits information in response to a request from the processor 30 to the external communication device via the network 16. In addition, the communication I/F 28 receives the information transmitted from the external communication device, and outputs the received information to the processor 30 via the bus 36.

A prediction processing program 32A is stored in the storage 32. The prediction processing program 32A is a program that provides a prediction simulation related to metabolism of a chemical substance. The processor 30 reads out the prediction processing program 32A from the storage 32 and executes the read-out prediction processing program 32A on the RAM 34 to perform metabolism prediction processing. The metabolism prediction processing is realized by the processor 30 operating as a first acquisition unit 30A and a metabolism prediction unit 30B.

In addition, an evaluation processing program 32B is stored in the storage 32. The evaluation processing program 32B is a program that provides an evaluation simulation of toxicity of a chemical substance. The processor 30 reads out the evaluation processing program 32B from the storage 32 and executes the read-out evaluation processing program 32B on the RAM 34 to perform toxicity evaluation processing. The toxicity evaluation processing is realized by the processor 30 operating as a second acquisition unit 30C and a toxicity evaluation unit 30D.

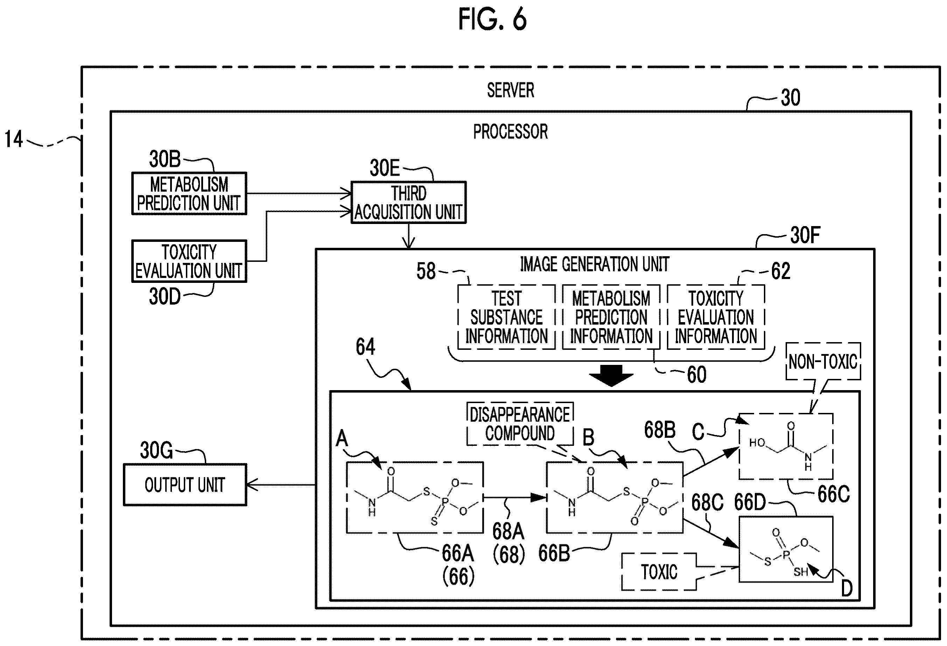

In addition, a generation processing program 32C is stored in the storage 32. The generation processing program 32C is a program that generates a display image (see FIG. 6) to be output to the client terminal 12. The processor 30 reads out the generation processing program 32C from the storage 32 and executes the read-out generation processing program 32C on the RAM 34 to perform image generation processing. The image generation processing is realized by the processor 30 operating as a third acquisition unit 30E, an image generation unit 30F, and an output unit 30G. The generation processing program 32C is an example of a "program" according to the technology of the present disclosure.

Here, a case where the user 18 uses the information processing system 10 to make a prediction regarding metabolism in a body (hereinafter, also simply referred to as a "metabolism prediction") and an evaluation of toxicity to a human body (hereinafter, also simply referred to as a "toxicity evaluation") for a newly developed chemical substance A will be considered. Here, the toxicity refers to toxicity that the user 18 is interested in as an evaluation item, and examples thereof include mutagenicity, sensitization, and irritation. The mutagenicity refers to a property of causing an irreversible change in genetic information (a base sequence of deoxyribonucleic acid (DNA) or a structure or number of chromosomes) of an organism. The sensitization refers to a property of causing an allergic reaction upon exposure to a chemical substance. The irritation refers to a property in which a chemical substance or the like gives a stimulus (for example, pain or a burning sensation) to the tactile sense or the like.

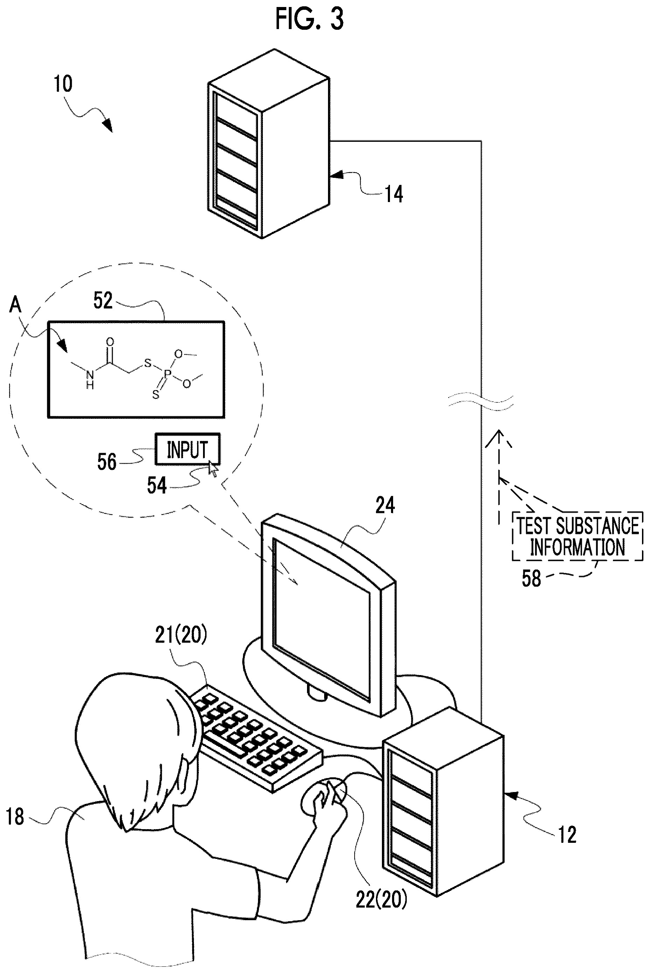

In a case of evaluating the toxicity, as shown in FIG. 3 as an example, the user 18 inputs data indicating a chemical substance A (hereinafter, also simply referred to as a "test substance A") that is a target of the metabolism prediction and the toxicity evaluation to the client terminal 12 via the reception device 20. Examples of the test substance include a low-molecular-weight compound (for example, a compound having a molecular weight of less than 1000), a sugar, a peptide, a protein, and a nucleic acid. The test substance A is an example of a "test substance" according to the technology of the present disclosure.

In the example shown in FIG. 3, a screen for selecting a test substance is displayed on a screen of the display device 24, and the user 18 performs a selection operation on the selection screen to select, for example, the test substance A. On the selection screen, for example, a test substance graph 52 is displayed, which shows a chemical structure of the test substance A as a compound graph (that is, a data structure in which atoms are nodes and bonds are edges). The user 18 clicks an input soft key 56 by operating a pointer 54 displayed on the screen of the display device 24 via the mouse 22. As a result, test substance information 58 is transmitted from the client terminal 12 to the server 14, and a processing request is made regarding the metabolism prediction and the toxicity evaluation for the test substance A.

Here, the test substance information 58 is information for specifying the chemical structure of the test substance A. The test substance graph 52 is, for example, image data, and the test substance information 58 is information composed of text information or numerical information representing a chemical structure of the test substance. The display device 24 reads out the test substance information 58 corresponding to the selected test substance graph 52 with reference to table data or the like in which a correspondence relationship between the test substance graph 52 and the test substance information 58 is recorded.

Here, although an example in which the compound graph is input as the data indicating the test substance A has been described, this is merely an example. The data input to the client terminal 12 need only be data for specifying the test substance A, and may be the test substance information 58. More specifically, the test substance information 58 is text information describing a chemical structure such as a structural formula, a compositional formula, and an amino acid sequence, or numerical information obtained by converting such text information into a numerical value. In addition, the data indicating the test substance A may be a chemical abstract service (CAS) number or a name of a chemical substance.

In addition, there may be a plurality of test substances A. In this case, for example, the user 18 may input data in which the plurality of test substances A are listed.

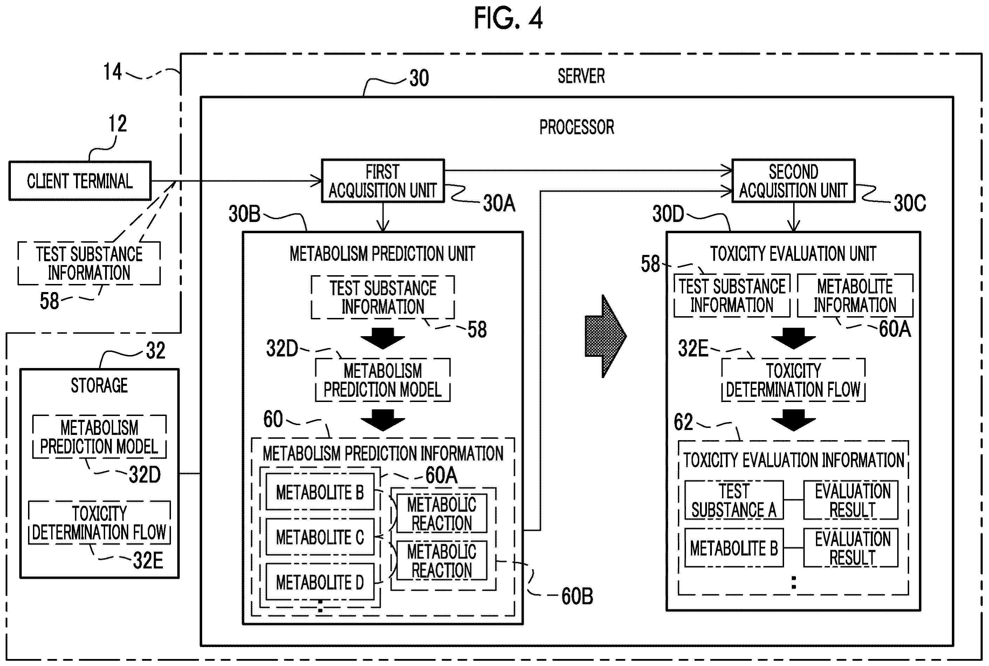

As shown in FIG. 4 as an example, the test substance information 58 is output from the client terminal 12 to the server 14. In the processor 30 of the server 14, the metabolism prediction processing is executed. The metabolism prediction processing is processing of predicting a metabolic reaction pathway of a test substance that occurs in a case where the test substance is subjected to a toxicity evaluation test and metabolites generated in the metabolic reaction pathway, and outputting the metabolic reaction pathway and the metabolites as a prediction result. Hereinafter, the test substance and the metabolite may be collectively referred to as simply "compound".

In the metabolism prediction processing, first, the first acquisition unit 30A acquires the test substance information 58. The first acquisition unit 30A outputs the test substance information 58 to the metabolism prediction unit 30B. The metabolism prediction unit 30B makes a prediction regarding the metabolism of the test substance A in the body, which is indicated by the test substance information 58. Here, the prediction regarding the metabolism includes a prediction of metabolic reactions that occur in a chain starting from the test substance A and a prediction of metabolites, which are compounds generated by the metabolic reactions.

Specifically, the metabolism prediction unit 30B acquires a metabolism prediction model 32D from the storage 32. The metabolism prediction model 32D is a model including rule data for predicting the metabolic reaction pathway and the metabolite. The processor 30 executes the metabolism prediction processing based on the test substance information 58 and the rule data. The rule data is data representing a rule in which a metabolic reaction proceeds. The rule data includes, for example, information on a metabolic reaction of a compound, that is, information on what kind of metabolic reaction a compound having what kind of structure generates and what kind of metabolite is generated. The processor 30 predicts the metabolic reaction and the metabolite while collating the test substance information 58 with the rule data. The metabolic reaction occurs not only in the test substance but also in the metabolite. Therefore, the metabolic reactions occur in a chain starting from the test substance. In a case where a metabolite is predicted by a metabolic reaction occurring in the test substance, the processor 30 also predicts a metabolic reaction occurring in the predicted metabolite. As described above, the processor 30 predicts metabolic reactions that occur in a chain in each compound of the test substance and the metabolite, and records the metabolic reactions as a metabolic reaction pathway.

Here, although an example of a form in which the prediction of the metabolic reaction and the metabolite is performed using the rule-based metabolism prediction model 32D using the rule data has been described, this is merely an example. The metabolism prediction model 32D may be, for example, a trained model for predicting a metabolic reaction using an artificial intelligence (AI) method. Such a trained model realizes a function of predicting a metabolic reaction, for example, by training a neural network through machine learning using training data. An example of training data is a data set obtained from results of past experiments, in which information for specifying a test substance is used as example data, and information for specifying a metabolite and information for specifying a metabolic reaction are used as correct answer data.

Then, the metabolism prediction unit 30B inputs the test substance information 58 to the metabolism prediction model 32D. The metabolism prediction model 32D outputs metabolism prediction information 60 corresponding to the input test substance information 58. The metabolism prediction unit 30B acquires the metabolism prediction information 60 output from the metabolism prediction model 32D. The metabolism prediction information 60 includes metabolic reaction information 60B that is information for specifying the predicted metabolic reaction, and metabolite information 60A that is information for specifying the metabolite generated by the metabolic reaction. The metabolite information 60A is information for specifying a chemical structure of a metabolite, which is a compound, as with the test substance information 58.

The first acquisition unit 30A outputs the test substance information 58 to the second acquisition unit 30C, and the metabolism prediction unit 30B outputs the metabolism prediction information 60 to the second acquisition unit 30C. After the metabolism prediction processing is executed by the processor 30, the processor 30 proceeds to the toxicity evaluation processing.

The toxicity evaluation processing is processing of evaluating toxicity of at least one compound selected from the group consisting of a test substance and metabolites. In the toxicity evaluation processing, the second acquisition unit 30C outputs the test substance information 58 and the metabolism prediction information 60 to the toxicity evaluation unit 30D. The toxicity evaluation unit 30D evaluates the toxicity of the test substance A indicated by the test substance information 58 and toxicity of the metabolite indicated by the metabolite information 60A.

Specifically, the toxicity evaluation unit 30D acquires a toxicity determination flow 32E from the storage 32. The toxicity determination flow 32E is described in the evaluation processing program 32B, and is schematically shown in a form of being stored in the storage 32 in FIG. 4. The toxicity determination flow 32E is a determination flow used for evaluating the toxicity. The toxicity determination flow 32E has at least one determination step, and has a route that branches according to a determination result in the determination step. Determination items in each determination step are predetermined. Examples of the determination item include whether or not a compound disappears through metabolism, whether or not a compound permeates a cell membrane, and whether or not a compound corresponds to a structural-alert rule. The structural-alert rule is a regulation related to a chemical structure, and is a rule for specifying a partial structure that may have toxicity of interest to the user.

The toxicity evaluation unit 30D inputs the test substance information 58 and the metabolite information 60A to the toxicity determination flow 32E. In the toxicity determination flow 32E, a determination regarding the toxicity evaluation is executed for any compound of the test substance or the metabolite in each determination step. The toxicity determination flow 32E is an example of a "determination flow" according to the technology of the present disclosure. Hereinafter, the test substance information 58 and the metabolite information 60A will be collectively referred to as compound information.

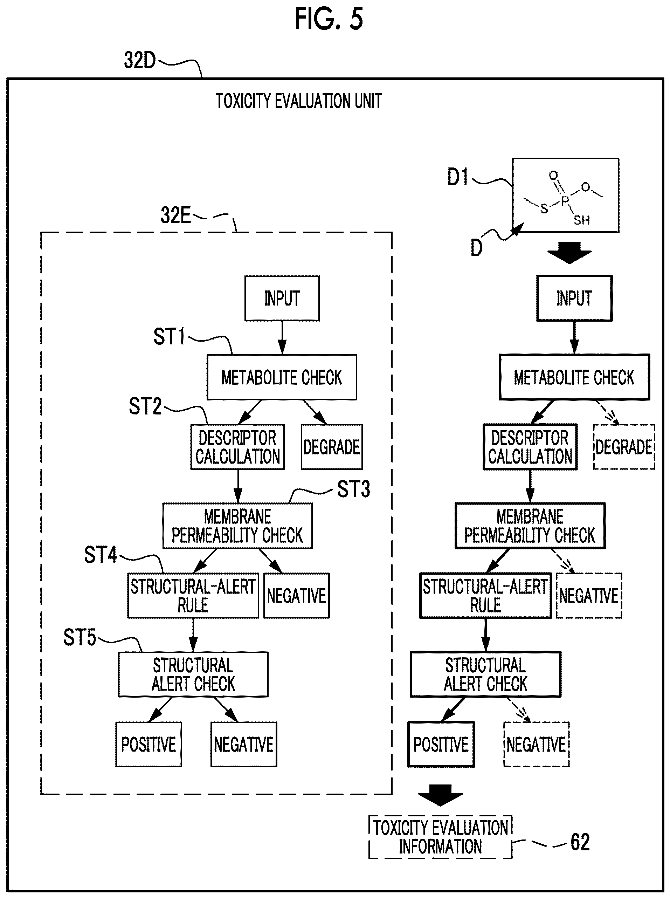

In the following description of the toxicity determination flow 32E, a metabolite D will be described as an example. As shown in FIG. 5 as an example, compound information corresponding to a compound graph D1 showing the metabolite D is input to the toxicity determination flow 32E. In the toxicity determination flow 32E, in step ST1, it is determined whether or not the metabolite D represented by the compound graph D1 is a compound that persists in the body without being metabolically degraded. In a case where the determination in step ST1 is affirmative, the toxicity evaluation for the metabolite D proceeds to step ST2. In a case where the determination in step ST1 is negative, as a result of the toxicity evaluation for the metabolite D, an evaluation result that the metabolite D is a compound (hereinafter, also simply referred to as a "disappearance compound") that is degraded and disappears in the body is output.

Here, in a metabolic reaction, a metabolic enzyme forms a complex with a compound that fits into an active site of the metabolic enzyme. The metabolic enzyme has an action of promoting a metabolic reaction by lowering the energy (that is, the activation energy) required for the compound to cause a chemical reaction. The term "disappearance compound" refers to a compound that, in a case of forming a complex with a metabolic enzyme, has lower activation energy than other metabolites, undergoes a chemical reaction more rapidly than other metabolites, and is converted into the next metabolite in a short period of time. Therefore, the disappearance compound is less likely to react with DNA in a cell, and the necessity of evaluating toxicity (that is, positive or negative evaluation) is low.

In step ST2, descriptor calculation is executed for the compound information corresponding to the compound graph D1. Examples of the descriptor include a molar mass or a LogP (a value obtained by taking the common logarithm of an octanol/water partition coefficient) of the metabolite D. After the process of step ST2 is executed, the toxicity evaluation proceeds to step ST3.

In step ST3, it is determined whether or not the compound can permeate the cell membrane based on a result of the descriptor calculation of the metabolite D executed in step ST2. The numerical value indicated by the descriptor is compared with a threshold value, and a determination is made as to whether or not the compound can permeate the cell membrane based on a comparison result. In a case where the determination in step ST3 is affirmative, the toxicity evaluation proceeds to step ST4. In a case where the determination in step ST3 is negative, a negative (that is, non-toxic) evaluation result is output as the result of the toxicity evaluation. This is because the compound does not permeate the cell membrane and is therefore considered to have little effect on the body.

In step ST4, the structural-alert rule is applied to the compound information corresponding to the compound graph D1. After the process of step ST4 is executed, the toxicity evaluation proceeds to step ST5.

In step ST5, it is determined whether or not a molecular structure indicated by the compound information corresponding to the compound graph D1 includes a structural alert. In a case where the determination in step ST5 is negative, a negative (that is, non-toxic) evaluation result is output as the result of the toxicity evaluation. This is because the metabolite D does not have a structural alert and is therefore considered to have a low likelihood of exhibiting toxicity.

In a case where the determination in step ST5 is affirmative, a positive (that is, toxic) evaluation result is output as the result of the toxicity evaluation. In the example shown in FIG. 5, paths and determination steps taken in the toxicity determination flow 32E are shown as the toxicity evaluation for the metabolite D, and an example in which the metabolite D is finally evaluated as positive is shown. As described above, the toxicity determination flow 32E outputs toxicity evaluation information 62 according to the input test substance information 58 and metabolite information 60A. In the toxicity evaluation information 62, the evaluation result is linked to each of the test substance A and its metabolites (see FIG. 4). In this way, the toxicity evaluation processing is executed.

Here, although an example of a form in which the toxicity evaluation processing is executed after the metabolism prediction processing is executed has been described, the technology of the present disclosure is not limited to this. The metabolism prediction processing and the toxicity evaluation processing may be executed in parallel. For example, during the metabolism prediction processing, the toxicity evaluation in the toxicity evaluation processing may be sequentially performed on each metabolite whose generation has been predicted.

As shown in FIG. 6 as an example, the metabolism prediction unit 30B outputs the metabolism prediction information 60 to the third acquisition unit 30E. In addition, the toxicity evaluation unit 30D outputs the test substance information 58 and the toxicity evaluation information 62 to the third acquisition unit 30E. The third acquisition unit 30E outputs the acquired test substance information 58, the acquired metabolism prediction information 60, and the acquired toxicity evaluation information 62 to the image generation unit 30F. The image generation unit 30F generates a display image 64 based on the test substance information 58, the metabolism prediction information 60, and the toxicity evaluation information 62. The display image 64 is an image showing the result of the prediction regarding the metabolism and the result of the toxicity evaluation for the compound. In addition, the display image 64 is an image showing the evaluation result of the toxicity of the compound in a distinguishable manner in a metabolic reaction pathway that systematically shows the metabolic reactions that occur in a chain starting from the test substance and the resulting metabolites. The display image 64 is an example of a "display image" according to the technology of the present disclosure.

In the example shown in FIG. 6, the display image 64 includes a compound image 66 showing a compound graph of each of the test substance A and the metabolites B to D. In addition, the display image 64 includes a connecting line 68 that connects the compound images 66 before and after the metabolic reaction. The metabolites B to D are examples of a "metabolite" according to the technology of the present disclosure, the compound image 66 is an example of "compound data" according to the technology of the present disclosure, and the connecting line 68 is an example of a "connecting line" according to the technology of the present disclosure.

Specifically, a compound image 66A showing a compound graph of the test substance A is shown in a left portion of the display image 64. In addition, a compound image 66B showing a compound graph of the metabolite B is shown in a central portion of the display image 64. A connecting line 68A is shown between the compound image 66A and the compound image 66B.

In addition, a compound image 66C showing a compound graph of the metabolite C is shown in an upper right portion of the display image 64. A connecting line 68B is shown between the compound image 66B and the compound image 66C. A compound image 66D showing a compound graph of the metabolite D is shown in a lower right portion of the display image 64. A connecting line 68C is shown between the compound image 66B and the compound image 66D. As described above, in the display image 64, the metabolic reaction pathway that systematically shows the metabolic reactions that occur in a chain starting from the test substance A and the resulting metabolites B to D is shown as a tree diagram.

In the display image 64, frame lines of the compound image 66A showing the test substance A and the compound image 66B showing the metabolite B are one-dot chain lines. This indicates that the test substance A and the metabolite B are disappearance compounds. In addition, in the display image 64, a frame line of the compound image 66C showing the metabolite C is a dotted line. This indicates that the evaluation result of the toxicity of the metabolite C is "non-toxic". Further, in the display image 64, a frame line of the compound image 66D showing the metabolite D is a solid line. This indicates that the evaluation result of the toxicity of the metabolite D is "toxic".

As described above, in the display image 64, the evaluation result of the toxicity of the compound is shown in a distinguishable manner. In addition, the display image 64 shows the compound image 66B showing the metabolite B, which is a disappearance compound, is shown, and shows that the metabolite B is a disappearance compound in a distinguishable manner. The image generation unit 30F outputs the generated display image 64 to the output unit 30G.

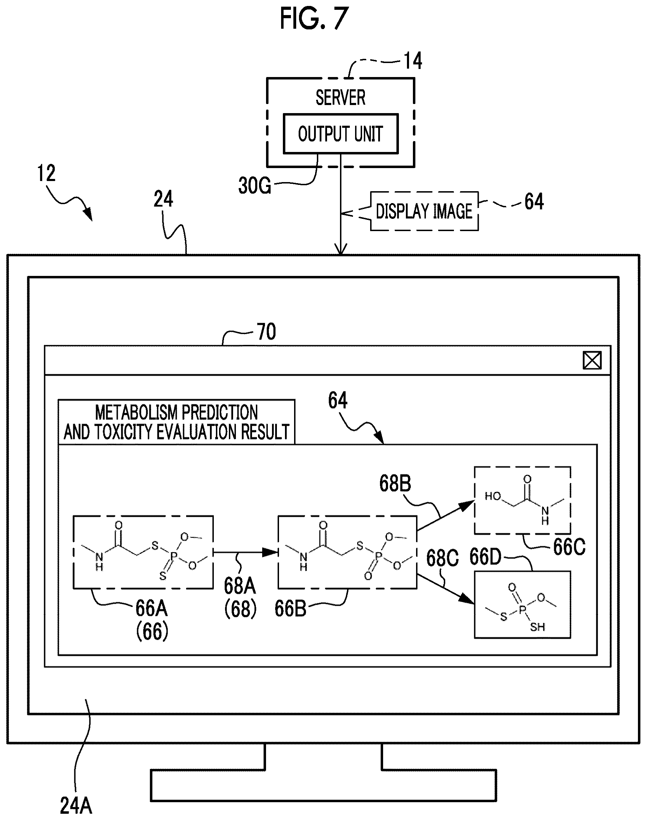

As shown in FIG. 7 as an example, the output unit 30G of the server 14 executes control of outputting the display image 64 to the client terminal 12. In other words, the server 14 transmits information indicating the display image 64 to the client terminal 12. On the display device 24 of the client terminal 12, a screen 24A including the display image 64 is displayed.

In the example shown in FIG. 7, the display image 64 is displayed in a window 70. As described above, in the display image 64, the metabolic reaction pathway that systematically shows the metabolic reactions that occur in a chain starting from the test substance A and the resulting metabolites B to D is shown as a tree diagram. In addition, in the display image 64, the evaluation result of the toxicity of the compound is shown in a distinguishable manner. The user 18 can understand the result of the metabolism prediction for the test substance A and the result of the toxicity evaluation of the test substance A and the metabolites B to D by visually recognizing the display image 64.

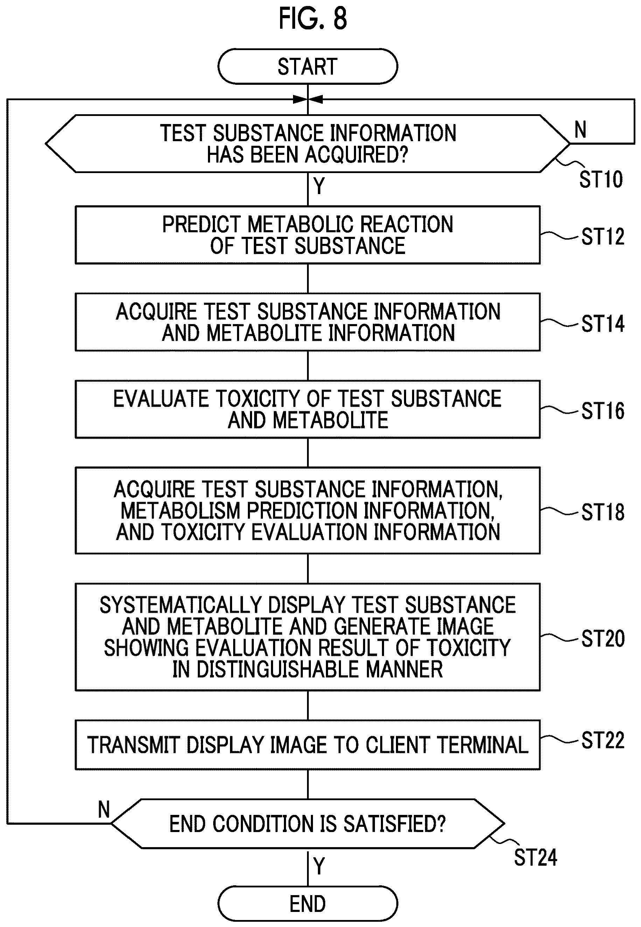

Next, control processing of the server 14 according to the present embodiment will be described with reference to FIG. 8. The server control processing is processing executed by the server 14, and includes the above-described metabolism prediction processing, toxicity evaluation processing, and image generation processing. FIG. 8 is a flowchart showing an example of the server control processing. The flow of the processing shown in FIG. 8 is an example of an "information processing method" according to the technology of the present disclosure.

In the control processing shown in FIG. 8 as an example, first, in step ST10, the first acquisition unit 30A determines whether or not the test substance information 58 has been acquired. In a case where it is determined in the determination in step ST10 that the test substance information 58 has been acquired, the determination is affirmative, and the server control processing proceeds to step ST12. In a case where it is determined in the determination in step ST10 that the test substance information 58 has not been acquired, the determination is negative, and the server control processing returns to step ST10.

In step ST12, the metabolism prediction unit 30B predicts a metabolic reaction based on the test substance information 58 acquired in step ST10. Specifically, the metabolism prediction unit 30B inputs the test substance information 58 to the metabolism prediction model 32D. The metabolism prediction model 32D outputs the metabolism prediction information 60 indicating a metabolite corresponding to the test substance A. The metabolism prediction unit 30B acquires the metabolism prediction information 60. After the process of step ST12 is executed, the server control processing proceeds to step ST14.

In step ST14, the second acquisition unit 30C acquires the test substance information 58 and the metabolite information 60A. After the process of step ST14 is executed, the server control processing proceeds to step ST16.

In step ST16, the toxicity evaluation unit 30D evaluates the toxicity of the test substance indicated by the test substance information 58 acquired in step ST14 and the toxicity of the metabolite indicated by the metabolite information 60A. Specifically, the toxicity evaluation unit 30D executes the toxicity evaluation of the test substance and the metabolite by using the toxicity determination flow 32E. The toxicity evaluation unit 30D acquires the toxicity evaluation information 62 which is information indicating the result of the toxicity evaluation of the test substance and the metabolite. After the process of step ST16 is executed, the server control processing proceeds to step ST18.

In step ST18, the third acquisition unit 30E acquires the test substance information 58, the metabolism prediction information 60, and the toxicity evaluation information 62. After the process of step ST18 is executed, the server control processing proceeds to step ST20.

In step ST20, the image generation unit 30F generates the display image 64 based on the test substance information 58, the metabolism prediction information 60, and the toxicity evaluation information 62 acquired in step ST18. The display image 64 is an image showing the evaluation result of the toxicity of the compound in a distinguishable manner in a metabolic reaction pathway that systematically shows the metabolic reactions that occur in a chain starting from the test substance and the resulting metabolites. In addition, the display image 64 includes an image showing a disappearance compound, and shows that the metabolite is a disappearance compound in a distinguishable manner. After the process of step ST20 is executed, the server control processing proceeds to step ST22.

In step ST22, the output unit 30G executes control of outputting the display image 64 generated by the image generation unit 30F in step ST20 to the client terminal 12. Specifically, the output unit 30G transmits the display image 64 to the client terminal 12. After the process of step ST22 is executed, the server control processing proceeds to step ST24.

In step ST24, the output unit 30G determines whether or not a condition (hereinafter, referred to as an "end condition") for ending the server control processing is satisfied. An example of the end condition is that the display image 64 has been transmitted to the client terminal 12. Another example of the end condition is that an instruction to end the server control processing has been accepted. In step ST24, in a case where the end condition is not satisfied, the determination is negative, and the server control processing proceeds to step ST10. In step ST24, in a case where the end condition is satisfied, the determination is affirmative, and the server control processing ends.

As described above, in the information processing system 10 according to the present embodiment, the display image 64 is generated by the processor 30 of the server 14. The display image 64 shows the prediction results of the metabolic reactions of the test substance A in the body and the metabolites B to D generated by the metabolic reactions, and the evaluation result of the toxicity of at least one compound in a compound group composed of the test substance A and the metabolites B to D. In addition, the display image 64 shows the evaluation result of the toxicity of the compound in a distinguishable manner in the metabolic reaction pathway that systematically shows the metabolic reactions that occur in a chain starting from the test substance A and the resulting metabolites B to D. By systematically displaying the metabolites B to D starting from the test substance A and also displaying the toxicity evaluation, it is possible to efficiently understand the prediction result of the metabolic reaction and the evaluation result of the toxicity of the compound involved in the metabolic reaction. Furthermore, by also displaying the metabolite B that disappears in the reaction pathway, the metabolic reaction pathway can be more easily understood. As a result, this configuration makes it possible to support the user in evaluating the toxicity of the compound.

For example, it is easier to understand which compound has toxicity in the metabolic reaction pathway, as compared with a case where the compounds are displayed in a list regardless of the reaction pathway. In addition, in a case where a disappearing metabolites are also displayed, the number of displayed metabolites is increased in a list display, making it difficult to see the display, but, in this configuration, the metabolites are displayed systematically, making it easier to understand the results of the metabolic prediction and the toxicity evaluation even in a case where there is a disappearing compound.

In addition, in the information processing system 10 according to the present embodiment, in the display image 64, the evaluation result of the metabolite B that disappears in the metabolic reaction pathway is displayed in a distinguishable manner from the other metabolites C and D. The metabolite B that disappears in the metabolic reaction pathway has a low necessity of being taken into consideration in the toxicity evaluation, as compared with the metabolites C and D that persist after the reaction. Therefore, by displaying the disappearing metabolite B in the metabolic reaction pathway in a distinguishable manner, the user can easily pay attention to the metabolite other than the disappearance compound, making it easier to understand the metabolic reaction pathway.

In addition, the information processing system 10 according to the present embodiment, the metabolic reaction pathway shown in the display image 64 is a tree diagram shown by the compound image 66 representing each compound of the test substance A and the metabolites B to D and the connecting lines 68 connecting the compound images 66 before and after the metabolic reaction. By presenting the metabolic reaction pathway as a tree diagram, it is easy to understand how the compound has changed during the metabolic reaction pathway. As a result, the metabolic reaction pathway becomes easier to understand.

In the present embodiment, although an example of a form in which the result of the toxicity evaluation shown in the display image 64 is distinguishable by the type of the frame line of the compound image 66 showing the compound has been described, the technology of the present disclosure is not limited to this. The display aspect of the result of the toxicity evaluation need only be an aspect in which the user 18 can distinguish the result of the toxicity evaluation need, and, for example, a color (for example, red for positive, green for negative, and gray for disappearance compound), a shape (for example, circular, triangular, or quadrangular), and/or a thickness of the frame line of the compound image 66 showing the compound may be changed. In addition, a text and/or a mark indicating the toxicity evaluation may be added to the compound image 66.

In addition, in the present embodiment, although an example of a form in which the result of the toxicity evaluation shown in the display image 64 is displayed for all compounds has been described, the technology of the present disclosure is not limited to this. For example, the result of the toxicity evaluation for some compounds (for example, the test substance A) does not need to be displayed.

In addition, in the present embodiment, although an example of a form in which the compounds are systematically displayed by being arranged from left to right in the display image 64 has been described, the technology of the present disclosure is not limited to this. In the display image 64, the compounds may be systematically displayed from right to left, or from top to bottom.

In addition, in the present embodiment, although an example of a form in which the connecting line 68 is an arrow in the display image 64 has been described, the technology of the present disclosure is not limited to this. The connecting line 68 need only be a line that connects the compound images 66 showing the compounds before and after the reaction, and the connecting line 68 may be a straight line or a curved line.

In addition, in the present embodiment, although an example of a form in which the metabolic reaction pathway is shown by the tree diagram in the display image 64 has been described, the technology of the present disclosure is not limited to this. In the display image 64, the metabolites need only be systematically shown starting from the test substance A, and the compound images 66 showing the compounds may be systematically arranged without the connecting line 68 therebetween.

In addition, in the present embodiment, although an example of a form in which the test substance A and the metabolites B to D are shown by the compound image 66 showing the compound graph in the display image 64 has been described, the technology of the present disclosure is not limited to this. In the display image 64, the user 18 need only recognize the test substance A and the metabolites B to D, and an image showing a CAS number, a substance name, or a structural formula may be displayed instead of or together with the compound image 66.

### (First Modification Example)

In the first embodiment, although an example of a form in which the connecting line 68 is displayed in the display image 64 has been described, the technology of the present disclosure is not limited to this. In the present first modification example, in the display image 64, a name of the metabolic reaction is displayed in association with the connecting line 68.

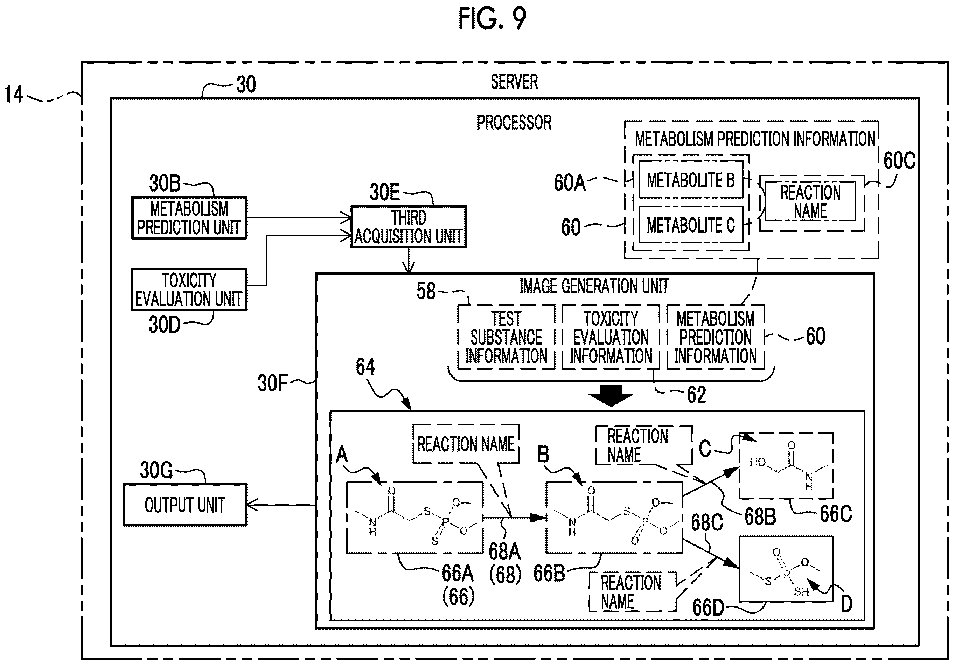

As shown in FIG. 9 as an example, in the processor 30 of the server 14, the third acquisition unit 30E acquires the test substance information 58, the metabolism prediction information 60, and the toxicity evaluation information 62. Here, the metabolism prediction information 60 includes the metabolite information 60A and reaction name information 60C. Here, the reaction name information 60C is information for specifying a name of the metabolic reaction. The reaction name information 60C is generated together with the metabolic reaction information 60B in the metabolism prediction processing. Then, the image generation unit 30F generates a display image 64 based on the test substance information 58, the metabolism prediction information 60, and the toxicity evaluation information 62.

In the example shown in FIG. 9, the display image 64 includes a compound image 66 showing a compound graph of each of the test substance A and the metabolites B to D. In addition, the display image 64 includes a connecting line 68 that connects the compound images 66 before and after the metabolic reaction. A reaction name of the metabolic reaction is associated with the connecting line 68 based on the reaction name information 60C.

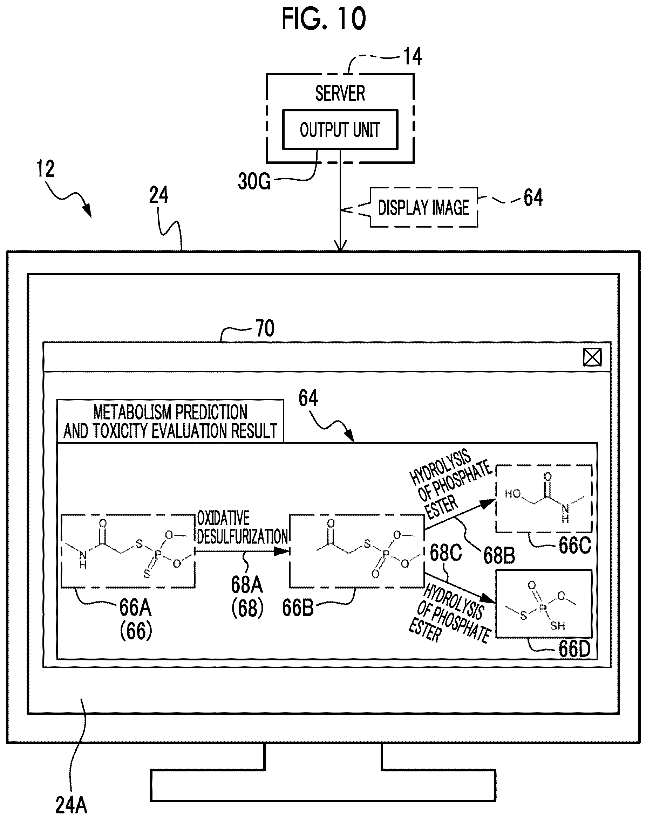

As shown in FIG. 10 as an example, the output unit 30G of the server 14 executes control of outputting the display image 64 to the client terminal 12. On the display device 24 of the client terminal 12, a screen 24A including the display image 64 is displayed.

In the example shown in FIG. 10, the display image 64 is displayed in the window 70. The display image 64 shows, as a tree diagram, the metabolic reaction pathway that systematically shows the metabolic reactions that occur in a chain starting from the test substance A and the resulting metabolites B to D, and shows the evaluation result of the toxicity of the compound in a distinguishable manner. In addition, in the display image 64, a text indicating the reaction name corresponding to the connecting line 68 is displayed.

In the example shown in FIG. 10, in the display image 64, a text "oxidative desulfurization" is displayed above the connecting line 68A. This indicates that test substance A is changed to the metabolite B by oxidative desulfurization. In addition, in the display image 64, a text of "hydrolysis of phosphate ester" is displayed above the connecting line 68B. This indicates that the metabolite B is changed to the metabolite C by hydrolysis of the phosphate ester. In addition, in the display image 64, a text of "hydrolysis of phosphate ester" is displayed below the connecting line 68C. This indicates that the metabolite B is changed to the metabolite D by hydrolysis of the phosphate ester. As described above, in the display image 64, the connecting line 68 and the reaction name are displayed in association with each other.

The user 18 can understand the result of the metabolism prediction and the result of the toxicity evaluation by visually recognizing the display image 64. In addition, the user 18 can recognize the name of the metabolic reaction by visually recognizing the display image 64.

As described above, in the information processing system 10 according to the present first modification example, in the processor 30 of the server 14, the image generation unit 30F generates the display image 64. The display image 64 includes the connecting line 68 that connects the compound images 66 before and after the metabolic reaction. Then, the name of the metabolic reaction is displayed in association with the connecting line 68. This makes it easier to understand what reactions have changed compounds during the metabolic reaction pathway. As a result, it becomes easier to understand the metabolic reaction pathway as compared with a case where the reaction name is not displayed.

In the first modification example, although an example of a form in which the reaction name is displayed in text in the display image 64 has been described, the technology of the present disclosure is not limited to this. It is sufficient that the user 18 can specify the metabolic reaction by visually recognizing the display image 64, and, in the display image 64, a mark indicating the reaction name may be displayed instead of or together with the text indicating the reaction name.

### (Second Modification Example)

In the first embodiment, although an example of a form in which the connecting line 68 is displayed in the display image 64 has been described, the technology of the present disclosure is not limited to this. In the present second modification example, in a case where the connecting line 68 is selected by the user 18 in the display image 64, a chemical structure that causes a metabolic reaction is displayed.

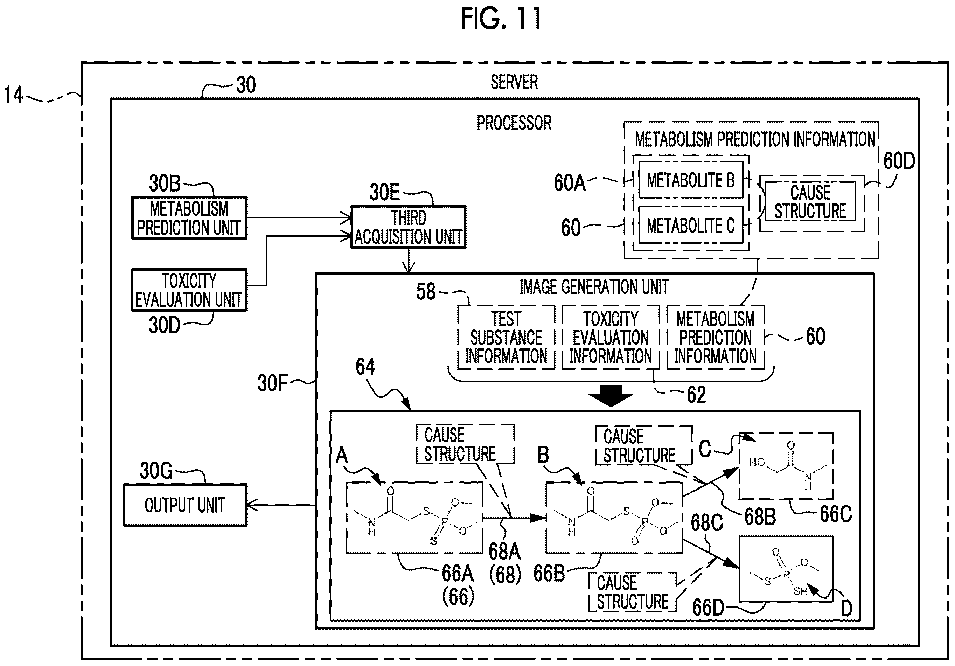

As shown in FIG. 11 as an example, in the processor 30 of the server 14, the third acquisition unit 30E acquires the test substance information 58, the metabolism prediction information 60, and the toxicity evaluation information 62. Here, the metabolism prediction information 60 includes the metabolite information 60A and cause structure information 60D. Here, the cause structure information 60D is information for specifying a chemical structure that causes a metabolic reaction, the chemical structure being a partial structure (hereinafter, also simply referred to as a "cause structure") contained in a compound before the reaction. The cause structure information 60D is generated together with the metabolic reaction information 60B in the metabolism prediction processing. Then, the image generation unit 30F generates a display image 64 based on the test substance information 58, the metabolism prediction information 60, and the toxicity evaluation information 62.

In the example shown in FIG. 11, the display image 64 includes a compound image 66 showing a compound graph of each of the test substance A and the metabolites B to D. In addition, the display image 64 includes a connecting line 68 that connects the compound images 66 before and after the metabolic reaction. The cause structure is associated with the connecting line 68 based on the cause structure information 60D.

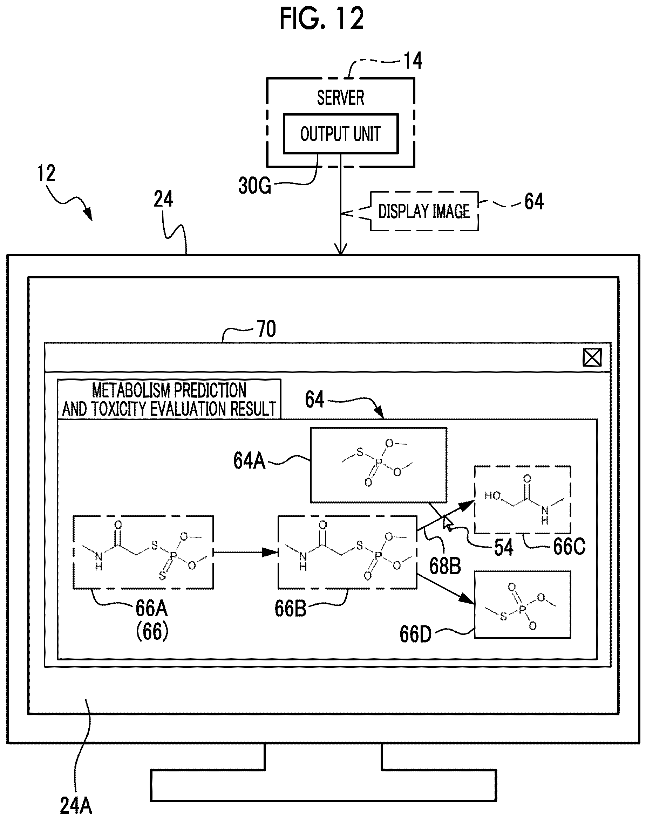

As shown in FIG. 12 as an example, the output unit 30G of the server 14 executes control of outputting the display image 64 to the client terminal 12. On the display device 24 of the client terminal 12, a screen 24A including the display image 64 is displayed.

In the example shown in FIG. 12, the display image 64 is displayed in the window 70. The display image 64 shows, as a tree diagram, the metabolic reaction pathway that systematically shows the metabolic reactions that occur in a chain starting from the test substance A and the resulting metabolites B to D, and shows the evaluation result of the toxicity of the compound in a distinguishable manner. In addition, in a case where the connecting line 68 is selected in the display image 64, the cause structure is displayed. In the example shown in FIG. 12, in a case where the user 18 selects the connecting line 68B via the pointer 54, a cause structure image 64A showing the cause structure in the reaction in which the metabolite B is changed to the metabolite C is popped up in the display image 64.

The user 18 can understand the result of the metabolism prediction and the result of the toxicity evaluation by visually recognizing the display image 64. In addition, the user 18 can recognize the cause structure by visually recognizing the cause structure image 64A.

As described above, in the information processing system 10 according to the present second modification example, in the processor 30 of the server 14, the image generation unit 30F generates the display image 64. The display image 64 includes the connecting line 68 that connects the compound images 66 before and after the metabolic reaction. Then, in a case where the connecting line 68 is selected, the cause structure image 64A is displayed. This makes it easier to understand what chemical structure caused the compound to change during the metabolic reaction pathway. As a result, the metabolic reaction pathway becomes easier to understand.

### (Third Modification Example)

In the first embodiment, although an example of a form in which the metabolic reaction pathway is displayed as it is in the display image 64 has been described, the technology of the present disclosure is not limited to this. In the present third modification example, in the display image 64, parts of the metabolic reaction pathway are collectively displayed.

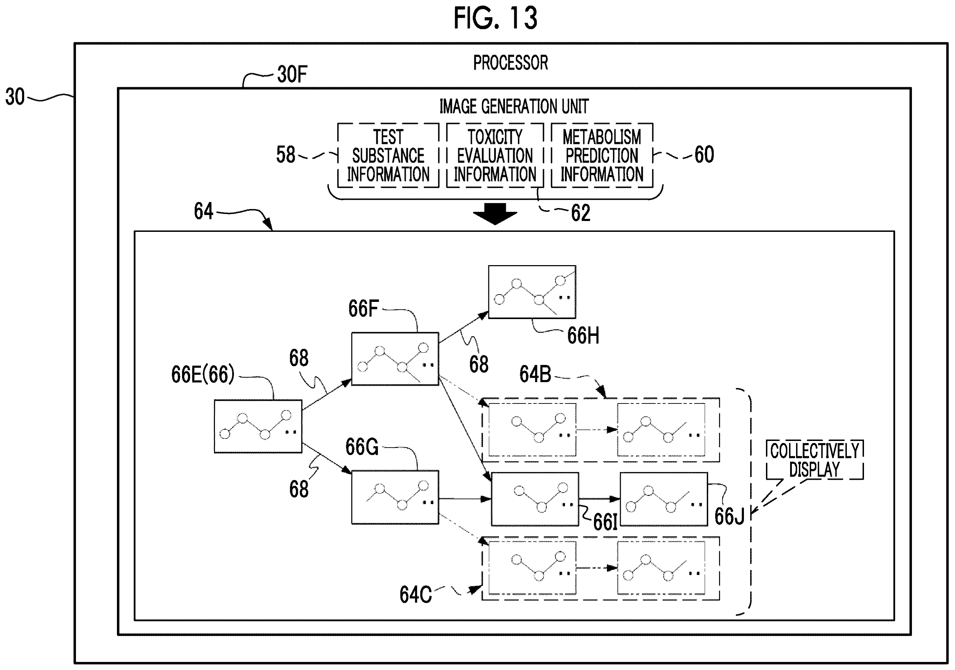

As shown in FIG. 13 as an example, in the processor 30, the image generation unit 30F generates a display image 64 based on the test substance information 58, the metabolism prediction information 60, and the toxicity evaluation information 62. In the example shown in FIG. 13, the display image 64 includes compound images 66E to 66J showing compounds. In addition, the display image 64 includes a connecting line 68 that connects the compound images 66 before and after the metabolic reaction. In the display image 64, a metabolic reaction pathway systematically shown by the compound images 66F to 66J is shown starting from the compound image 66E showing the test substance.

In the metabolic reaction pathways shown in the display image 64 of FIG. 13, partial metabolic reaction pathways originating from different metabolites are shared. In this case, the partial metabolic reaction pathways are displayed collectively. In the example shown in FIG. 13, a partial pathway image 64B showing a partial metabolic reaction pathway that changes in the order of the compound image 66I and the compound image 66J starting from the metabolite indicated by the compound image 66F is shown. In addition, a partial pathway image 64C showing a partial metabolic reaction pathway that changes in the order of the compound image 66I and the compound image 66J starting from the metabolite indicated by the compound image 66G is shown. The partial pathway images 64B and 64C share a reaction pathway and are therefore collectively displayed as one.

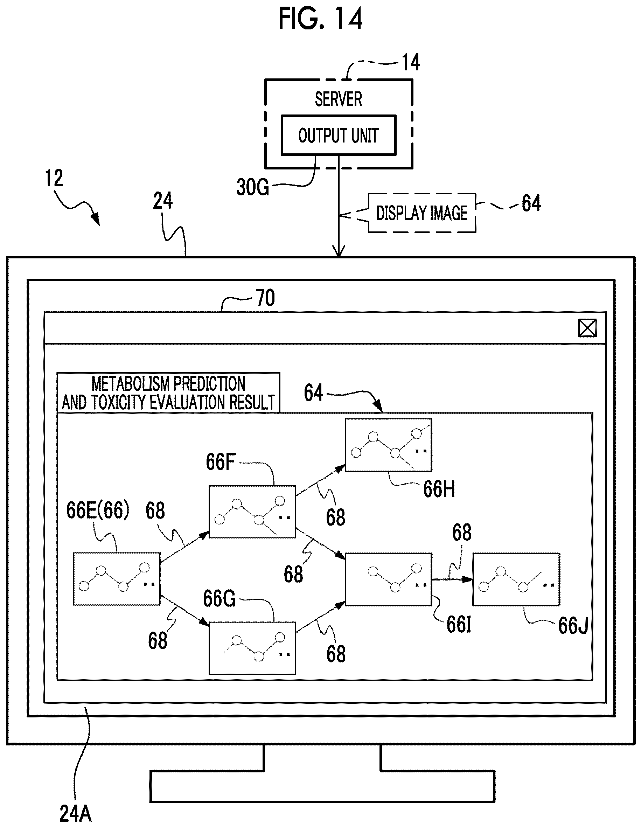

As shown in FIG. 14 as an example, the output unit 30G of the server 14 executes control of outputting the display image 64 to the client terminal 12. On the display device 24 of the client terminal 12, a screen 24A including the display image 64 is displayed.

In the example shown in FIG. 14, the display image 64 is displayed in the window 70. In the display image 64, the metabolic reaction pathway is shown as a tree diagram, and the evaluation result of the toxicity of the compound is shown in a distinguishable manner. In addition, in the display image 64, the shared partial metabolic reaction pathways (here, the reaction pathway in which the metabolite indicated by the compound image 66I is changed to the metabolite indicated by the compound image 66J) are collectively displayed. The user 18 can understand the result of the metabolism prediction and the result of the toxicity evaluation by visually recognizing the display image 64.

As described above, in the information processing system 10 according to the present third modification example, in the metabolic reaction pathway, in a case where the partial metabolic reaction pathways originating from a plurality of different metabolites are shared, the partial metabolic reaction pathways are collectively displayed as one. As a result, parts that have shared partial metabolic reaction pathways are displayed collectively, making it easier to view the entire display image 64 and also making it possible to reduce the display range of the display image 64.

### (Fourth Modification Example)

In the first embodiment, although an example of a form in which the metabolic reaction pathway is displayed in the display image 64 has been described, the technology of the present disclosure is not limited to this. In the present fourth modification example, in the display image 64, the toxicity determination flow 32E used in the toxicity evaluation is displayed together with the metabolic reaction pathway.

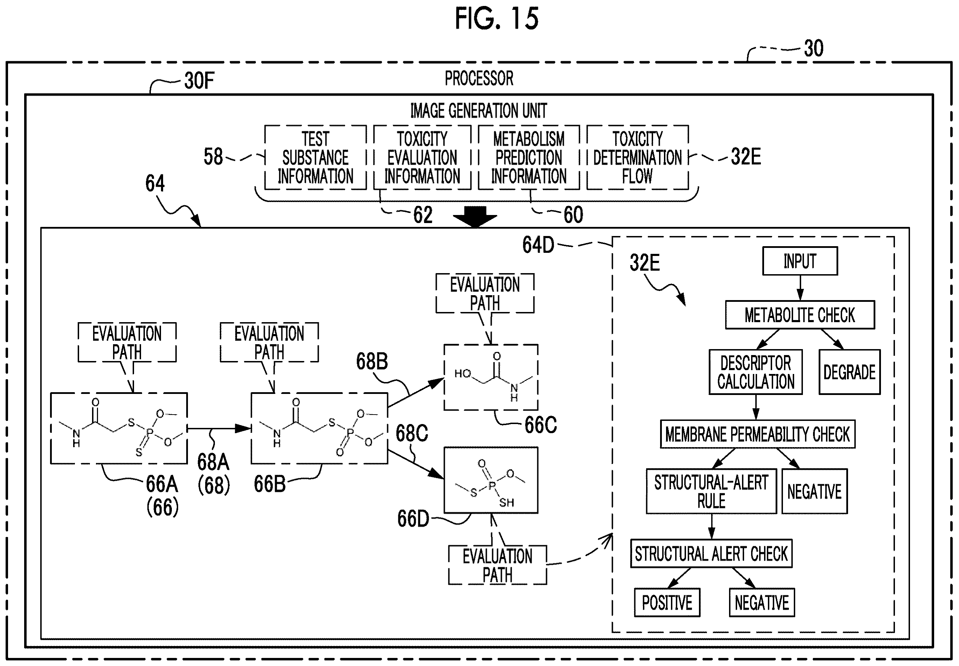

As shown in FIG. 15 as an example, in the processor 30, the image generation unit 30F generates a display image 64 based on the test substance information 58, the metabolism prediction information 60, the toxicity evaluation information 62, and the toxicity determination flow 32E.

In the example shown in FIG. 15, the display image 64 includes a compound image 66 showing a compound graph of each of the test substance A and the metabolites B to D. In addition, the display image 64 includes a connecting line 68 that connects the compound images 66 before and after the metabolic reaction. Further, the display image 64 includes a flow image 64D showing the toxicity determination flow 32E. In the present modification example, the toxicity evaluation information 62 includes information indicating the result of the toxicity evaluation as well as information indicating a path (hereinafter, also simply referred to as an "evaluation path") leading to the result of the toxicity evaluation in the toxicity determination flow 32E.

In the display image 64, the compound indicated by the compound image 66 and the evaluation path of the toxicity determination flow 32E are associated with each other.

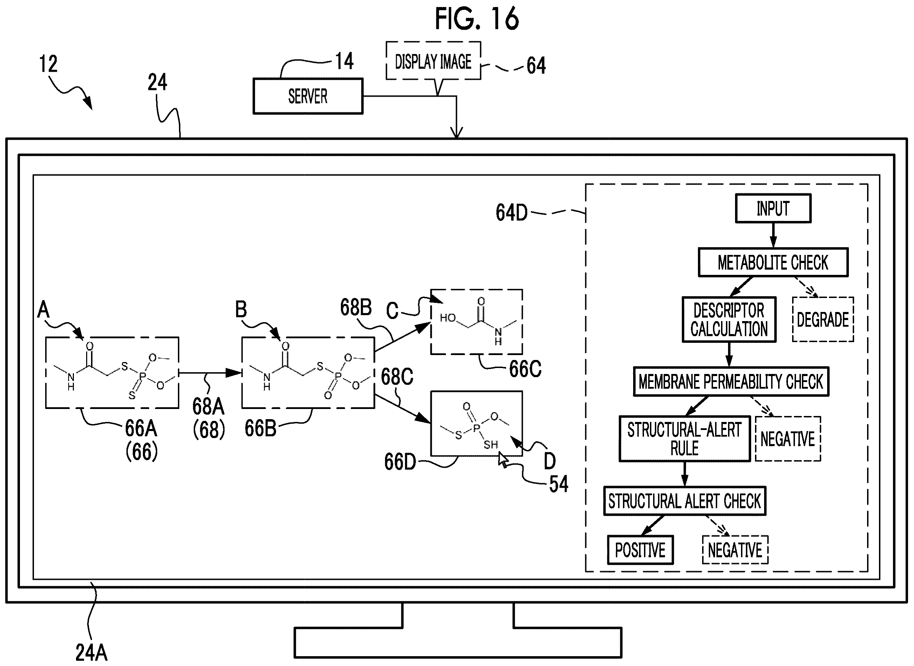

As shown in FIG. 16 as an example, the server 14 executes control of outputting the display image 64 to the client terminal 12. On the display device 24 of the client terminal 12, a screen 24A including the display image 64 is displayed. The display image 64 shows, as a tree diagram, the metabolic reaction pathway that systematically shows the metabolic reactions that occur in a chain starting from the test substance A and the resulting metabolites B to D, and shows the evaluation result of the toxicity of the compound in a distinguishable manner.

In addition, in a case where the compound image 66 showing the compound is selected in the display image 64, an evaluation path is displayed in the flow image 64D. In the example shown in FIG. 16, in a case where the compound image 66D is selected by the user 18 via the pointer 54, an evaluation path of the metabolite D indicated by the compound image 66D (here, a path leading to "positive") is displayed in the flow image 64D. In addition, in the flow image 64D, the evaluation path is shown to be distinguishable from other paths in the toxicity determination flow 32E. In the example shown in FIG. 16, the evaluation path is indicated by a thick line, and the other paths are indicated by a broken line.

Here, the evaluation path is displayed to be distinguishable from other paths by changing the line type indicating the evaluation path, but this is merely an example. The evaluation path may be distinguishable from other paths by changing a color of the evaluation path, or the evaluation path may be distinguishable from other paths by surrounding the evaluation path with a frame line.

The user 18 can understand the result of the metabolism prediction and the result of the toxicity evaluation by visually recognizing the display image 64. In addition, the user 18 can recognize the evaluation path by visually recognizing the flow image 64D.

As described above, in the information processing system 10 according to the present fourth modification example, in a case where the compound image 66 showing the compound is selected in the display image 64, the flow image 64D is displayed in which, among a plurality of paths in the toxicity determination flow 32E, an evaluation path of the selected compound is shown in a distinguishable manner from the other paths. This makes it easier to understand which evaluation path has been followed for the toxicity evaluation.

### (Fifth Modification Example)

In the first embodiment, although an example of a form in which the metabolic reaction pathway is displayed as it is in the display image 64 has been described, the technology of the present disclosure is not limited to this. In the present fifth modification example, in the display image 64, the display range of the metabolic reaction pathway can be adjusted.

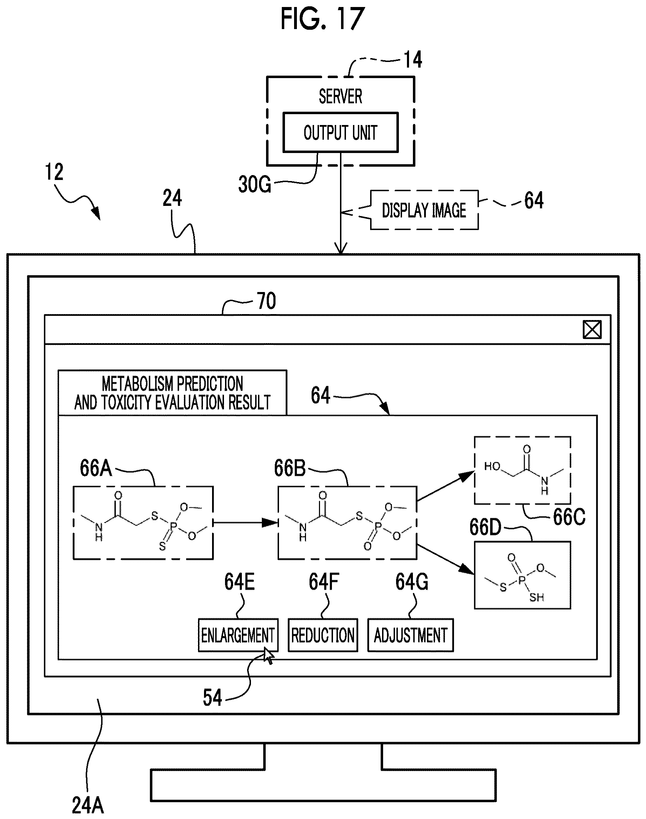

As shown in FIG. 17 as an example, the server 14 executes control of outputting the display image 64 to the client terminal 12. As a result, the display image 64 is displayed on the screen 24A of the display device 24. An enlargement soft key 64E, a reduction soft key 64F, and an adjustment soft key 64G are displayed in a lower part of the display image 64.

The enlargement soft key 64E is a soft key for enlarging the display magnification of the metabolic reaction pathway, and the reduction soft key 64F is a soft key for reducing the display magnification of the metabolic reaction pathway. The user 18 presses the enlargement soft key 64E or the reduction soft key 64F via the pointer 54 to increase or decrease the display magnification of the metabolic reaction pathway. In addition, the adjustment soft key 64G is a soft key for restoring the display range of the metabolic reaction pathway to the original range. The user 18 presses the adjustment soft key 64G via the pointer 54 to return the display range of the metabolic reaction pathway to a state before the change in the display range. As described above, in the display image 64, the display range of the metabolic reaction pathway can be adjusted.

As described above, in the information processing system 10 according to the present fifth modification example, in the display image 64, the display range of the metabolic reaction pathway can be adjusted. By adjusting the display range of the metabolic reaction pathway, it becomes easier to understand the metabolic reaction pathway in a case where the metabolic reaction pathway is complex (for example, in a case where the metabolic reaction pathway branches multiple times and many metabolites are generated).

In the fifth modification example, although an example of a form in which the display range is enlarged or reduced by the enlargement soft key 64E or the reduction soft key 64F has been described, the technology of the present disclosure is not limited to this. For example, in a case where the mouse 22 as the reception device 20 comprises a wheel, the display image 64 may be enlarged or reduced by operating the wheel.

### [Second Embodiment]

In the first embodiment, although an example of a form in which the metabolism prediction processing, the toxicity evaluation processing, and the image generation processing are performed in the server 14 has been described, the technology of the present disclosure is not limited to this. In the present second embodiment, the metabolism prediction processing and the toxicity evaluation processing are executed in the server 14, and the image generation processing is executed in the client terminal 12. In the present embodiment, the client terminal 12 is an example of an "information processing apparatus" according to the technology of the present disclosure.

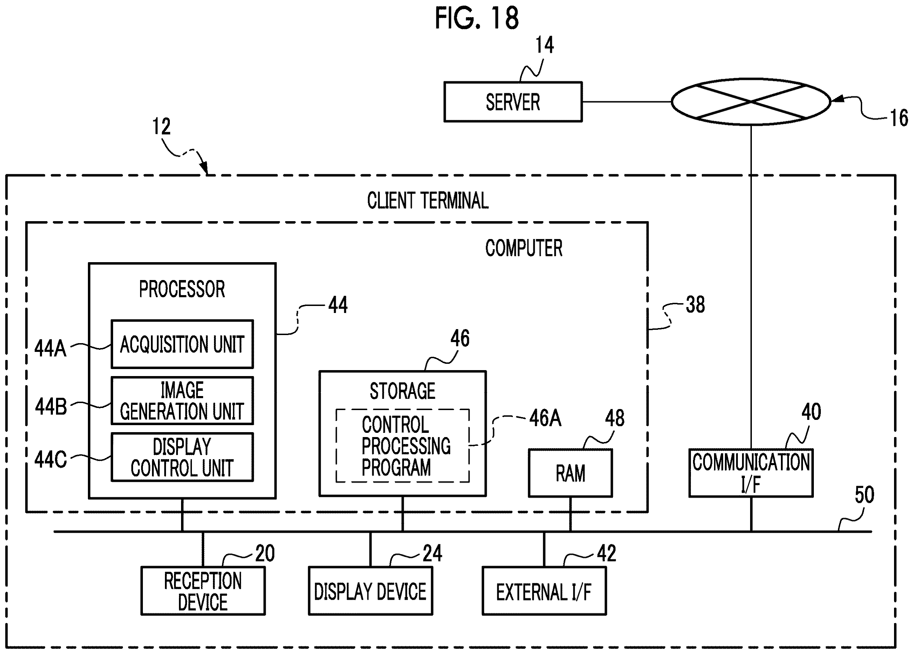

As shown in FIG. 18 as an example, the client terminal 12 comprises a computer 38, the reception device 20, the display device 24, a communication I/F 40, an external I/F 42, and a bus 50. The computer 38 comprises a processor 44, a storage 46, and a RAM 48. The processor 44, the storage 46, the RAM 48, the reception device 20, the display device 24, the communication I/F 40, and the external I/F 42 are connected to the bus 50.

In addition, since a hardware configuration (that is, the processor 44, the storage 46, and the RAM 48) of the computer 38 is basically the same as the hardware configuration of the computer 26, a description of the hardware configuration of the computer 38 will be omitted here.

The communication I/F 40 is connected to the network 16. The communication I/F 40 is responsible for transmitting and receiving information to and from an external communication device (for example, the server 14) via the network 16. For example, the communication I/F 40 transmits information in response to a request from the processor 44 to the external communication device via the network 16. In addition, the communication I/F 40 receives the information transmitted from the external communication device, and outputs the received information to the processor 44 via the bus 50.

The external I/F 42 is responsible for transmitting and receiving various types of information to and from an external device (not shown) present outside the client terminal 12. The external device may be, for example, at least one of a smart device, a personal computer, a server, a universal serial bus (USB) memory, a memory card, or a printer. An example of the external I/F 42 is a USB interface. The external device is connected directly or indirectly to the USB interface.

A control processing program 46A is stored in the storage 46. The control processing program 46A is a program for executing display control of an image. The processor 30 executes display control processing by reading out the control processing program 46A from the storage 46 and executing the read-out control processing program 46A on the RAM 48. The display control processing is realized by the processor 44 operating as an acquisition unit 44A, an image generation unit 44B, and a display control unit 44C. The computer 38 is an example of a "computer" according to the technology of the present disclosure, and the control processing program 46A is an example of a "program" according to the technology of the present disclosure.

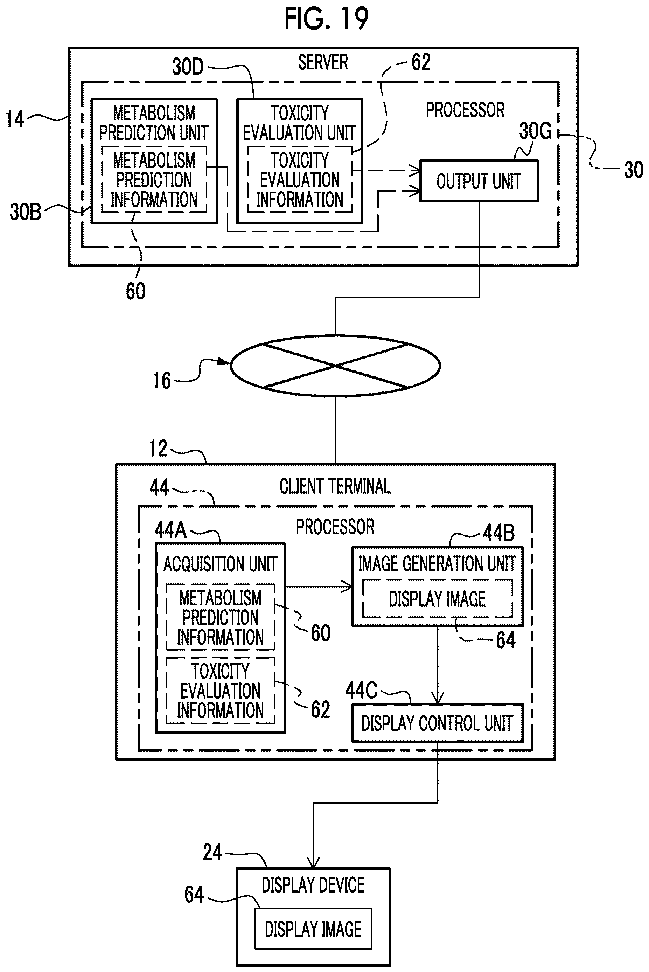

As shown in FIG. 19 as an example, in the processor 30 of the server 14, the metabolism prediction unit 30B outputs the metabolism prediction information 60 obtained by the metabolism prediction processing to the output unit 30G. In addition, the toxicity evaluation unit 30D outputs the toxicity evaluation information 62 obtained by the toxicity evaluation processing to the output unit 30G. The output unit 30G outputs the metabolism prediction information 60 and the toxicity evaluation information 62 to the client terminal 12 via the network 16.

In the processor 44 of the client terminal 12, the acquisition unit 44A acquires the metabolism prediction information 60 and the toxicity evaluation information 62 via the network 16. Then, the acquisition unit 44A outputs the metabolism prediction information 60 and the toxicity evaluation information 62 to the image generation unit 44B. The image generation unit 44B generates a display image 64 based on the metabolism prediction information 60 and the toxicity evaluation information 62. Then, the image generation unit 44B outputs the generated display image 64 to the display control unit 44C. The display control unit 44C performs graphical user interface (GUI) control for displaying the display image 64, thereby causing the display device 24 to display the display image 64. The user 18 can understand the result of the metabolism prediction for the test substance A and the result of the toxicity evaluation of the test substance A and the metabolites B to D by visually recognizing the display image 64.

### [Third Embodiment]

In the first embodiment, although an example of a form in which the metabolism prediction processing, the toxicity evaluation processing, and the image generation processing are performed in the server 14 has been described, the technology of the present disclosure is not limited to this. In the present third embodiment, the metabolism prediction processing, the toxicity evaluation processing, and the image generation processing are performed in the client terminal 12. In the present embodiment, the client terminal 12 is an example of an "information processing apparatus" according to the technology of the present disclosure.

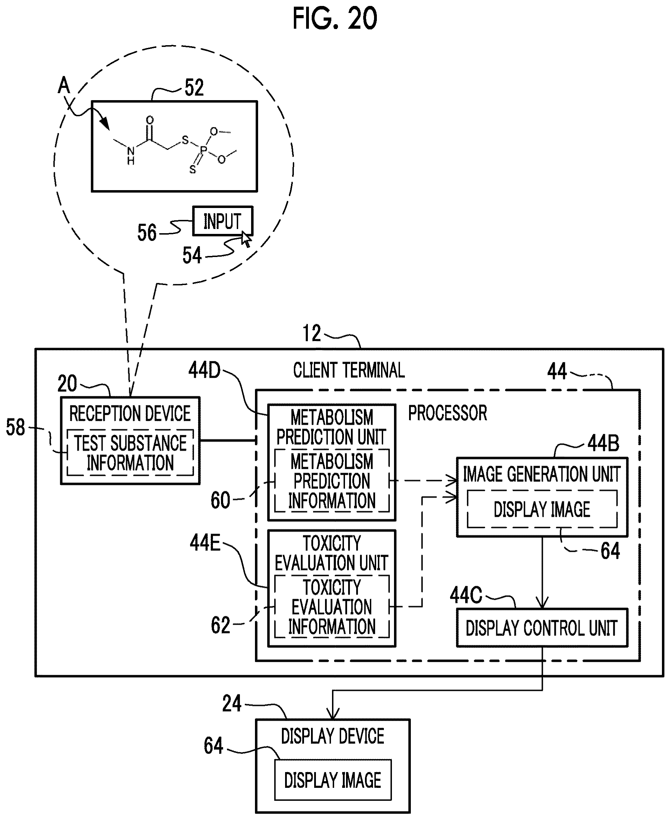

As shown in FIG. 20 as an example, the test substance information 58 is received in the client terminal 12 via the reception device 20. In the processor 44 of the client terminal 12, a metabolism prediction unit 44D performs the metabolism prediction of the test substance A based on the test substance information 58. In addition, a toxicity evaluation unit 44E performs the toxicity evaluation on the test substance A and the metabolites B to D. Then, the image generation unit 44B generates a display image 64 based on the metabolism prediction information 60 and the toxicity evaluation information 62. The display control unit 44C causes the display device 24 to display the display image 64. The user 18 can understand the result of the metabolism prediction for the test substance A and the result of the toxicity evaluation of the test substance A and the metabolites B to D by visually recognizing the display image 64.

In addition, in each of the above-described embodiments, although an example of a form in which the metabolism prediction information 60 and the toxicity evaluation information 62 are obtained by executing the metabolism prediction processing and the toxicity evaluation processing has been described, the technology of the present disclosure is not limited to this. For example, the server 14 or the client terminal 12 may receive already obtained metabolism prediction information 60 and toxicity evaluation information 62 (for example, metabolism prediction information 60 and toxicity evaluation information 62 obtained as a result of processing by an external device, or metabolism prediction information 60 and toxicity evaluation information 62 obtained in the past), and perform the image generation processing.

In addition, in each of the above-described embodiments, although an example of a form in which the prediction processing program 32A, the evaluation processing program 32B, and the generation processing program 32C are stored in the storage 32 has been described, the technology of the present disclosure is not limited to this. For example, the prediction processing program 32A, the evaluation processing program 32B, and the generation processing program 32C may be stored in a portable storage medium such as an SSD or a USB memory. The storage medium is a non-transitory computer-readable storage medium. The prediction processing program 32A, the evaluation processing program 32B, and the generation processing program 32C stored in the storage medium are installed in the computer 26. The processor 30 executes control processing of the server 14 in accordance with the prediction processing program 32A, the evaluation processing program 32B, and the generation processing program 32C. Similarly, the control processing program 46A may be stored in a storage medium instead of the storage 46.

In each of the above-described embodiments, the computers 26 and 38 are exemplified, but the technology of the present disclosure is not limited to this, and a device including an application specific integrated circuit (ASIC), a field programmable gate array (FPGA), and/or a programmable logic device (PLD) may be applied instead of the computers 26 and 38. In addition, instead of the computers 26 and 38, a hardware configuration and a software configuration may be used in combination.

The following various processors can be used as hardware resources for executing the various kinds of processing described in each of the above-described embodiments. Examples of the processor include a CPU that is a general-purpose processor that functions as a hardware resource for executing the software, that is, the program to execute the metabolism prediction processing, the toxicity evaluation processing, and/or the image generation processing (hereinafter, also simply referred to as "various kinds of processing"). Examples of the processor also include a dedicated electronic circuit that is a processor whose dedicated circuit configuration is specially designed to execute specific processing, such as an FPGA, a PLD, or an ASIC. Any processor includes a memory built therein or connected thereto, and any processor uses the memory to execute various kinds of processing.

The hardware resource for executing the various kinds of processing may be configured by one of the various processors or by a combination of two or more processors of the same type or different types (for example, a combination of a plurality of FPGAs or a combination of a processor and an FPGA). Further, the hardware resource for executing the various kinds of processing may be one processor.

A first example of the configuration in which the hardware resource is configured by one processor is an aspect in which one processor is configured by a combination of one or more processors and software, and this processor functions as the hardware resource for executing the various kinds of processing. Second, as represented by system-on-a-chip (SoC), there is a form in which a processor that realizes the functions of the entire system including a plurality of hardware resources for executing the various kinds of processing with a single integrated circuit (IC) chip. As described above, the various kinds of processing are implemented by using one or more of the various processors as the hardware resource.

Further, specifically, an electronic circuit in which circuit elements, such as semiconductor elements, are combined can be used as the hardware structure of these various processors. The various kinds of processing are merely examples. Accordingly, it is possible to delete an unnecessary step, add a new step, or change a processing order without departing from the gist of the present disclosure.

The content of the above description and the content of the drawings are detailed explanations of the parts relating to the technology of the present disclosure, and are merely examples of the technology of the present disclosure. For example, description related to the above configurations, functions, actions, and effects is description related to an example of configurations, functions, actions, and effects of the parts relating to the technology of the present disclosure. Thus, it is needless to say that unnecessary portions may be deleted, new elements may be added, or replacement may be made to the content of the above description and the content of the drawings without departing from the gist of the technology of the present disclosure. In order to avoid complication and easily understand the parts relating to the technology of the present disclosure, in the content of the above description and the content of the drawings, the description regarding common general technical knowledge which is not necessarily particularly described in terms of embodying the technology of the present disclosure is omitted.

In the present specification, "A and/or B" is synonymous with "at least one of A or B". That is, "A and/or B" may refer to A alone, B alone, or a combination of A and B. In addition, in the present specification, in a case in which three or more matters are expressed with the connection of "and/or", the same concept as "A and/or B" is applied.

All documents, patent applications, and technical standards mentioned in the present specification are incorporated herein by reference to the same extent as in a case in which each document, each patent application, and each technical standard are specifically and individually described by being incorporated by reference.

The disclosure of JP2023-059422 filed on March 31, 2023 is incorporated herein by reference in its entirety.

In regard to the embodiments described above, the following appendices will be further disclosed.

### <Appendix 1>

An information processing apparatus comprising:
a processor,
in which the processor is configured to
   generate a display image that shows a prediction result of metabolic reactions occurring in vivo from a test substance and metabolites generated by the metabolic reactions and an evaluation result of toxicity of at least one compound in a compound group composed of the test substance and the metabolites, the display image showing the evaluation result of the compound in a distinguishable manner in a metabolic reaction pathway that systematically shows the metabolic reactions occurring in a chain starting from the test substance, and the resulting metabolites, and
   execute control of outputting the generated display image, and
the metabolites systematically shown in the display image include a disappearance compound that disappears in the metabolic reaction pathway.

### <Appendix 2>

The information processing apparatus according to Appendix 1,
in which the evaluation result of the disappearance compound is displayed in the display image in a distinguishable manner from other metabolites.

### <Appendix 3>

The information processing apparatus according to Appendix 1 or 2,
in which the metabolic reaction pathway shown in the display image is a tree diagram shown by compound data representing each compound of the test substance and the metabolites and connecting lines connecting items of the compound data before and after the metabolic reaction.

### <Appendix 4>

The information processing apparatus according to Appendix 3,
in which a name of the metabolic reaction is displayed in association with the connecting line.

### <Appendix 5>

The information processing apparatus according to Appendix 3 or 4,
in which, in a case where the connecting line is selected, a chemical structure that causes the metabolic reaction is displayed, the chemical structure being a partial structure contained in the compound before the reaction.

### <Appendix 6>

The information processing apparatus according to any one of Appendices 1 to 5,
in which, in the metabolic reaction pathway, in a case where partial metabolic reaction pathways originating from a plurality of the metabolites different from each other are shared, the partial metabolic reaction pathways are collectively displayed as one.

### <Appendix 7>

The information processing apparatus according to any one of Appendices 1 to 6,
in which, in a case where the compound, which is a target of an evaluation of the toxicity and has the evaluation result, is selected in the metabolic reaction pathway in the display image, a determination flow indicating a toxicity determination procedure, which is used in the evaluation of the toxicity, has at least one determination step, and has a path that branches according to a determination result of the determination step, is displayed, in which, among a plurality of paths, a path that leads to the evaluation result of the compound is shown in a distinguishable manner from the other paths.

### <Appendix 8>

The information processing apparatus according to any one of Appendices 1 to 7,
in which, in the display image, a display range of the metabolic reaction pathway is adjustable.

## Claims

1. An information processing apparatus comprising:
a processor,
wherein the processor is configured to
generate a display image that shows a prediction result of metabolic reactions occurring in vivo from a test substance and metabolites generated by the metabolic reactions and an evaluation result of toxicity of at least one compound in a compound group composed of the test substance and the metabolites, the display image showing the evaluation result of the compound in a distinguishable manner in a metabolic reaction pathway that systematically shows the metabolic reactions occurring in a chain starting from the test substance, and the resulting metabolites, and
execute control of outputting the generated display image, and
the metabolites systematically shown in the display image include a disappearance compound that disappears in the metabolic reaction pathway.

2. The information processing apparatus according to claim 1,
wherein the evaluation result of the disappearance compound is displayed in the display image in a distinguishable manner from other metabolites.

3. The information processing apparatus according to claim 1,
wherein the metabolic reaction pathway shown in the display image is a tree diagram shown by compound data representing each compound of the test substance and the metabolites and connecting lines connecting items of the compound data before and after the metabolic reaction.

4. The information processing apparatus according to claim 3,
wherein a name of the metabolic reaction is displayed in association with the connecting line.

5. The information processing apparatus according to claim 3,
wherein, in a case where the connecting line is selected, a chemical structure that causes the metabolic reaction is displayed, the chemical structure being a partial structure contained in the compound before the reaction.

6. The information processing apparatus according to claim 1,
wherein, in the metabolic reaction pathway, in a case where partial metabolic reaction pathways originating from a plurality of the metabolites different from each other are shared, the partial metabolic reaction pathways are collectively displayed as one.

7. The information processing apparatus according to claim 1,
wherein, in a case where the compound, which is a target of an evaluation of the toxicity and has the evaluation result, is selected in the metabolic reaction pathway in the display image, a determination flow indicating a toxicity determination procedure, which is used in the evaluation of the toxicity, has at least one determination step, and has a path that branches according to a determination result of the determination step, is displayed, in which, among a plurality of paths, a path that leads to the evaluation result of the compound is shown in a distinguishable manner from the other paths.

8. The information processing apparatus according to claim 1,
wherein, in the display image, a display range of the metabolic reaction pathway is adjustable.

9. An information processing method comprising:
generating a display image that shows a prediction result of metabolic reactions occurring in vivo from a test substance and metabolites generated by the metabolic reactions and an evaluation result of toxicity of at least one compound in a compound group composed of the test substance and the metabolites, the display image showing the evaluation result of the compound in a distinguishable manner in a metabolic reaction pathway that systematically shows the metabolic reactions occurring in a chain starting from the test substance, and the resulting metabolites; and
executing control of outputting the generated display image,
wherein the metabolites systematically shown in the display image include a disappearance compound that disappears in the metabolic reaction pathway.

10. A program for causing a computer to execute a process comprising:
generating a display image that shows a prediction result of metabolic reactions occurring in vivo from a test substance and metabolites generated by the metabolic reactions and an evaluation result of toxicity of at least one compound in a compound group composed of the test substance and the metabolites, the display image showing the evaluation result of the compound in a distinguishable manner in a metabolic reaction pathway that systematically shows the metabolic reactions occurring in a chain starting from the test substance, and the resulting metabolites; and
executing control of outputting the generated display image,
wherein the metabolites systematically shown in the display image include a disappearance compound that disappears in the metabolic reaction pathway.
